# EUROPEAN PATENT SPECIFICATION

(11) **EP 3 563 594 B1**
(45) Date of publication and mention of the grant of the patent: **13.08.2025**
(21) Application number: 17889073.7
(22) Date of filing: 22.12.2017
(51) Int. Cl.: H04L 67/12, G16H 40/20, H04W 4/029, H04W 4/02, H04W 8/24, H04L 67/52

(54) **REAL TIME LOCATION PLATFORM BEACON PROTOCOL SYSTEMS AND METHODS**
BAKENPROTOKOLLSYSTEME UND -VERFAHREN FÜR ECHTZEITLOKALISIERUNGSPLATTFORM
SYSTÈMES ET PROCÉDÉS DE PROTOCOLE DE BALISE DE PLATEFORME DE LOCALISATION EN TEMPS RÉEL

(30) Priority: 31.12.2016 US 201662441233 P
(43) Date of publication of application: 06.11.2019
(73) Proprietor: GE Precision Healthcare LLC, Waukesha, Wisconsin 53188-1615 (US)
(72) Inventor: CANNELL, Matthew James, Glen Allen, Virginia 23059 (US); CRAWLEY, Philip, Glen Allen, Virginia 23059 (US); REED, Shawn, Glen Allen, Virginia 23059 (US)
(74) Representative: Fennell, Gareth Charles
(86) International application number: PCT/US2017/068117
(87) International publication number: WO 2018/125785

(56) References cited:
- KR-A- 20140 069 306
- US-A1- 2006 270 421
- US-A1- 2013 257 614
- US-A1- 2016 029 160
- US-A1- 2016 142 868
- US-A1- 2016 260 301
- US-A1- 2016 260 301
- US-B1- 8 949 022

## Description

### FIELD OF THE DISCLOSURE

This disclosure relates generally to healthcare environments, and, more particularly, to methods and apparatus to facilitate proximity detection and location tracking.

### BACKGROUND

Real-time location systems (RTLS) monitor asset distribution and usage, providing actionable information to help control costs and improve the quality and efficiency of care. Systems that have been developed to track and analyze activities in clinical settings have included installing Radio Frequency Identification (RFID) or infrared (IR) reader infrastructures into buildings to capture position information. RFID sensors may be placed on the people and/or assets that need to be tracked.

However, this is an expensive and time-consuming solution because it requires pulling power and data cabling to all the required locations. Location accuracy can also vary depending on technology. Typical RFID systems have a tolerance of approximately plus-or-minus ten feet, further limiting their range. RFID and IR-based sensors, though, are highly susceptible to drift due to interference in the environment (e.g., a patient room) and cross talk between locations that are physically separated, but have a line of sight between them (e.g., two patient rooms across the hall from each other).

Therefore, it would be desirable to design a system and method for tracking locations and interactions between people and assets in an environment with minimal infrastructure requirements and standardized technologies.
US 2016/0260301 discloses an asset tag device including a housing, a battery disposed within the housing, a circuit board disposed within the housing and in electrical communication with the battery. The circuit board includes a local memory, a transceiver to detect local signals and transmit a response packet, and a processor. The processor to identify the detected local signals, determine whether the detected local signals include at least one beacon signal, store, to the local memory, the at least one beacon signal, sort, in the local memory, the stored at least one beacon signal by proximity to the asset tag device, parse the sorted at least one beacon signal, and append tag information to the parsed at least one beacon signal to generate the response packet.
US 2006/270421 Al describes, according to its abstract, methods and systems relating to location-based services such as social networking, providing demographic information, tracking mobile devices, providing business information, providing an adaptable user interface, remotely effecting a change on a portable electronic device, providing a geofence, outputting location-based information on a mobile device, varying transmissions to and from a mobile device, providing location-based alerts, verifying transactions and tailoring information to the behavior of a user.
US 8 949 022 B1 discloses an electronic device configured to attach to a human-powered vehicle.

### BRIEF DESCRIPTION OF THE DRAWINGS

The features and technical aspects of the system and method disclosed herein will become apparent in the following Detailed Description set forth below when taken in conjunction with the drawings in which like reference numerals indicate identical or functionally similar elements.
FIG. 1 shows a block diagram of an example healthcare-focused information system.
FIG. 2 shows a block diagram of an example healthcare information infrastructure including one or more systems.
FIG. 3 shows an example industrial internet configuration including a plurality of health-focused systems.
FIG. 4 is a block diagram illustrating an example environment constructed in accordance with the teachings of this disclosure to facilitate proximity detection and location tracking.
FIG. 5 is a block diagram of the example reader badge of the example environment of FIG. 4.
FIG. 6 illustrates an example environment illustrating interaction between premises via a cloud.
FIG. 7 illustrates an example architecture of the hospital network and the cloud of the example of FIG. 6.
FIG. 8 illustrates a basic real time location platform including a number of monitored devices and an edge device in a facility, along with a cloud health processor and management service(s) in a cloud.
FIG. 9 illustrates an example implementation of the cloud health processor of FIG. 8.
FIG. 10 illustrates a flow diagram of an example method to monitor receiver and/or other device health in a real time location system.
FIG. 11 is a block diagram of an example server structured to execute the example machine-readable instructions of FIG. 10 to implement the example systems of FIGS. 1-9.

### DETAILED DESCRIPTION

In the following detailed description, reference is made to the accompanying drawings that form a part hereof, and in which is shown by way of illustration specific examples that may be practiced. These examples are described in sufficient detail to enable one skilled in the art to practice the subject matter, and it is to be understood that other examples may be utilized and that logical, mechanical, electrical and other changes may be made without departing from the scope of the subject matter of this disclosure. The following detailed description is, therefore, provided to describe an exemplary implementation and not to be taken as limiting on the scope of the subject matter described in this disclosure. Certain features from different aspects of the following description may be combined to form yet new aspects of the subject matter discussed below.

When introducing elements of various embodiments of the present disclosure, the articles "a," "an," "the," and "said" are intended to mean that there are one or more of the elements. The terms "comprising," "including," and "having" are intended to be inclusive and mean that there may be additional elements other than the listed elements.

### I. Overview

Certain examples of the presently disclosed technology improve proximity detection and location tracking of resources in an environment such as a hospital. An example system disclosed herein includes one or more beacon tags affixed to assets within the environment and that transmit (e.g., periodically, aperiodically and/or as a one-time event) beacon messages. The beacon messages are received by a mobile reader badge that listens for beacon messages transmitted in the environment. For example, disclosed example reader badges (sometimes referred to herein as "readers," "badges" or "mobile wireless bridges") may include a network interface to receive beacon messages transmitted via low power Bluetooth Low Energy (BLE). In some disclosed examples, the reader badges process the received beacon messages and communicate information obtained from the beacon messages to one or more real-time location services (RTLS) servers via a communication infrastructure. For example, disclosed example reader badges may aggregate and communicate a batch of beacon messages (e.g., a threshold number of beacon messages, a threshold interval of time (e.g., a window of interest), etc.) to an RTLS server via a Wi-Fi infrastructure (e.g., a wireless network). In some disclosed examples, the RTLS server processes the received batch of beacon messages to facilitate real-time location tracking of the resources in the environment. In some disclosed examples, the RTLS server may report the location of resources via charts, graphs, tables, etc.

Real-time location services enable improved patient workflow via proximity detection and location tracking in a healthcare environment, such as a hospital. Location tracking can be used to locate resources such as mobile assets (e.g., patients, intravenous (IV) pumps, telemetry units, wheelchairs, etc.) within the hospital. For example, location tracking can be used to locate a "lost" or "missing" IV pump within a patient's room. Proximity detection facilitates an improved understanding of how interactions occur during the patient workflow. For example, based on the proximity to a soap dispenser, a user (e.g., a system administrator) can determine whether a caretaker washed their hands prior to interacting with a patient.

Examples systems and methods disclosed herein facilitate improved proximity detection and location tracking by creating a hospital tracking network within the hospital using the communication infrastructure already installed in the hospital. Beacon tags are installed throughout a location or building. For example, beacon tags can be affixed to stationary assets (e.g., patient room entry ways, sinks, water fountains, hallways, etc.) and mobile assets such as hospital beds, IV pumps, soap dispensers, etc. In some disclosed examples, the beacon tags are also included in disposable patient tags provided to the patient upon admission of a hospital stay. Beacon tags are low-cost, low-power transmitters of beacon messages. A beacon message (sometimes referred to herein as a "beacon") includes information about the beacon tag such as a unique identifier (e.g., a tag identifier such as a media access control (MAC) address) and a tag type identifier (e.g., whether the beacon tag is affixed to a fixed-location asset or to a mobile asset). In some disclosed examples, the beacon tags broadcast (e.g., advertise, communicate, transmit, etc.) beacon messages at preset frequencies (e.g., ten times a second, once a second, once a minute, etc.). For example, a beacon tag affixed to a fixed-location asset (e.g., a sink) may broadcast beacon messages ten times a second, while a beacon tag affixed to a mobile asset (e.g., a wheelchair) may broadcast beacon messages at relatively shorter intervals (e.g., once a second).

A reader badge is a mobile wireless bridge that facilitates mobile tracking by "listening" and receiving beacon messages broadcast by beacon tags. The reader badge includes a BLE controller to receive connection-less beacon messages broadcast by beacon tags. The reader badge also includes a Wi-Fi controller to establish a connection with an RTLS server. The reader badge may be worn or transported by hospital caregivers. For example, a reader badge may be worn as a lanyard or clipped to the caregiver's clothing. As the caregiver moves about the hospital, the reader badge passively collects beacon messages and communicates reader messages to an RTLS server at the backend of the system. In some examples, the reader badge collects a number (e.g., a predetermined number) of beacon messages or waits a period (e.g., a predetermined period of time) prior to communicating the reader messages. In some examples, the reader badge generates and communicates a reader message as a beacon message from a beacon tag is received. A reader message includes information received from the beacon message such as a unique identifier of the source beacon tag and a spatial location of the source beacon tag. In some examples, the reader badge includes a timestamp identifying when the beacon message was received by the reader badge in the reader message. In some examples, the reader badge includes a received signal strength indication (RSSI) value (e.g., a power ratio in decibels of the measured power to one milli-watt (dBm)).

Example reader badges disclosed herein include a proximity engine to process the beacon messages and determine distance from the source (e.g., the beacon tag that broadcast the corresponding beacon message). For example, a hospital room may include a first beacon tag affixed to a door, a second beacon tag affixed to an infusion pump, a third beacon tag affixed to a bed, and a fourth beacon tag included in a patient tag (e.g., a disposable bracelet including patient identification information such as name, sex, date of birth information). As the caregiver moves about the hospital room, the reader badge may receive beacon messages from each of the beacon tags. The proximity engine can determine the RSSI strength for each of the beacon messages and associate RSSI strength with a respective beacon tag.

In some examples, the proximity engine determines which beacon tags are proximate (e.g., near or closely located) to the reader badge. For example, the proximity engine can compare the RSSI strength of a beacon message to a threshold and if the RSSI strength satisfies the threshold (e.g., the RSSI strength is greater than a threshold), the proximity engine identifies the source beacon tag as proximate to the reader badge. In some examples, the proximity engine discards beacon messages that are not proximate to the reader badge.

Example systems and methods disclosed herein include an RTLS server that monitors and/or reports tracking location and interactions between people and assets in an environment. For example, the RTLS server can aggregate reader messages from the one or more reader badges included in an environment (e.g., the hospital). The RTLS server may be in connection with the reader badges via a wireless Intranet network (e.g., a wireless local area network, etc.) and/or a wireless Internet connection.

As healthcare assets continue to become smaller and more ergonomic, RTLS tracking with a small footprint becomes increasingly important. Additionally, as a hospital's inventory of healthcare equipment gets leaner, the equipment is likely to be cleaned more often. Therefore, an asset tracking beacon should withstand frequent, repeated cleaning with harsh disinfecting chemicals.

Certain examples provide an improved housing that can be applied with BLE and/or other location tracking technology to healthcare assets (e.g., scanner, IV pumps, monitors, etc.). In certain examples, a computerized maintenance management system (CMMS) and/or source system can organize and monitor assets and can remove and reassociate beacons from one asset to another asset on demand. Beacons can be installed on ergonomic items that do not have flat surfaces. Beacons can be developed with housings to withstand rigorous healthcare cleaning protocols while maintaining a small footprint to not disturb normal usage of equipment to which the beacon is applied.

A quality of location data provided by a real time location platform can depend on health of devices deployed to receive sensory and/or location events. If deployed devices are not functioning as intended, the location data produced by the system may be inaccurate/unreliable. To help ensure accurate location data, support teams can monitor system health, isolate problematic devices and correct the problems through reconfiguration/replacement/upgrades/etc.

Certain examples provide receiver health methods and systems for real time location platforms. Certain examples define a mechanism and associated application programming interface (API) specification by which location receivers deployed as part of a real time location platform can transmit system health information using an event-based messaging framework. The data/events provided can be captured and utilized to maintain the system and help ensure improved or optimal performance.

Devices used to implement a real time location platform may have numerous dependencies, including a reliable power supply (e.g., battery, outlet, etc.), network connectivity and acceptable environmental conditions (e.g. min/max operating temperature, etc.). With a large number of devices deployed, it is not feasible or cost effective to manually inspect each device in the field on a regular basis. Certain examples facilitate device self-reporting of health status and associated system events to help maintain a functioning system.

In certain examples, location devices are designed to submit event data (e.g., as JavaScript Object Notation (JSON) documents, etc.) to a service interface (e.g., a representational state transfer (REST) or RESTful service interface, etc.). There are numerous events defined, and these events can be sent in response to a condition (e.g., device regaining network connectivity, device placed on charger, device removed from charger, etc.) or on a time schedule that is configurable as part of the device profile. Events include a set of base (e.g., header, etc.) attributes that are used for ongoing system health management. In addition, each event includes a details section where attributes/data specific to an event type can be included.

In certain examples, receiver health includes a set of events defined for receiver devices (e.g., Bluetooth receiver devices, BLE receiver devices, etc.). The set of events can be defined according to an API, for example. In certain examples, a gateway client API includes a service interface specification or API for the RESTful service used by the device to post receiver health events, etc.

Certain examples provide a centralized health and monitoring capability for large scale systems that include a plurality of devices deployed in a wide range of environments. Without such monitoring, deployed systems may fall into disrepair over time and/or the costs of monitoring/maintaining such systems may threaten the commercial viability of the dependent products, for example.

Certain examples, when utilized, result in improved system performance, higher customer satisfaction, higher return on investment for the customer, lower cost of ownership for the customer, lower support costs for the supplier and increased profit margin for the supplier, etc.

Types and details of health events reported by devices can be extended/modified in a variety of ways to propose a "unique" set of health events. The mechanisms/protocols by which the events are delivered (e.g. JSON/XML/CSV or HTTP/JMS/SMTP, etc.) and/or captured can also be varied to propose a "unique" solution, for example.

Certain examples provide a custom beacon protocol (e.g., a custom BLE beacon protocol, etc.) that provides all involved fields for message processing and location determination, as well as system health status including low battery indication (e.g., in days remaining, hours remaining, percent remaining, battery value, etc.), custom fields for additional installation specific data (e.g., profile identifier (ID), floor designation, department ID, etc.), beacon security, and/or other item(s). While no single existing protocol fulfills these needs, certain examples provide a new protocol that enable location systems to provide customers with system health and security information/messaging and also enable additional device level functionality by allowing custom data within the protocol.

### II. Example Operating Environment

Health information, also referred to as healthcare information and/or healthcare data, relates to information generated and/or used by a healthcare entity. Health information can include reader messages and RTLS server information, for example. Health information can be information associated with health of one or more patients, for example. Health information may include protected health information (PHI), as outlined in the Health Insurance Portability and Accountability Act (HIPAA), which is identifiable as associated with a particular patient and is protected from unauthorized disclosure. Health information can be organized as internal information and external information. Internal information includes patient encounter information (e.g., patient-specific data, aggregate data, comparative data, etc.) and general healthcare operations information, etc. External information includes comparative data, expert and/or knowledge-based data, etc. Information can have both a clinical (e.g., diagnosis, treatment, prevention, etc.) purpose and an administrative (e.g., scheduling, billing, management, etc.) purpose.

Institutions, such as healthcare institutions, having complex network support environments and sometimes chaotically driven process flows utilize secure handling and safeguarding of the flow of sensitive information (e.g., personal privacy). A need for secure handling and safeguarding of information increases as a demand for flexibility, volume, and speed of exchange of such information grows. For example, healthcare institutions provide enhanced control and safeguarding of the exchange and storage of sensitive patient PHI and employee information between diverse locations to improve hospital operational efficiency in an operational environment typically having a chaotic-driven demand by patients for hospital services. In certain examples, patient identifying information can be masked or even stripped from certain data depending upon where the data is stored and who has access to that data. In some examples, PHI that has been "de-identified" can be re-identified based on a key and/or other encoder/decoder.

A healthcare information technology infrastructure can be adapted to service multiple business interests while providing clinical information and services. Such an infrastructure may include a centralized capability including, for example, a data repository, reporting, discreet data exchange/connectivity, "smart" algorithms, personalization/consumer decision support, etc. This centralized capability provides information and functionality to a plurality of users including medical devices, electronic records, access portals, pay for performance (P4P), chronic disease models, and clinical health information exchange/regional health information organization (HIE/RHIO), and/or enterprise pharmaceutical studies, home health, for example.

Interconnection of multiple data sources helps enable an engagement of all relevant members of a patient's care team and helps improve an administrative and management burden on the patient for managing his or her care. Particularly, interconnecting the patient's electronic medical record and/or other medical data can help improve patient care and management of patient information. Furthermore, patient care compliance is facilitated by providing tools that automatically adapt to the specific and changing health conditions of the patient and provide comprehensive education and compliance tools to drive positive health outcomes.

In certain examples, healthcare information can be distributed among multiple applications using a variety of database and storage technologies and data formats. To provide a common interface and access to data residing across these applications, a connectivity framework (CF) can be provided which leverages common data models (CDM) and common service models (CSM) and service oriented technologies, such as an enterprise service bus (ESB) to provide access to the data.

In certain examples, a variety of user interface frameworks and technologies can be used to build applications for health information systems including, but not limited to, MICROSOFT^{®} ASP.NET, AJAX^{®}, MICROSOFT^{®} Windows Presentation Foundation, GOOGLE^{®} Web Toolkit, MICROSOFT^{®} Silverlight, ADOBE^{®}, and others. Applications can be composed from libraries of information widgets to display multi-content and multimedia information, for example. In addition, the framework enables users to tailor layout of applications and interact with underlying data.

In certain examples, an advanced Service-Oriented Architecture (SOA) with a modern technology stack helps provide robust interoperability, reliability, and performance. Example SOA includes a three-fold interoperability strategy including a central repository (e.g., a central repository built from Health Level Seven (HL7) transactions), services for working in federated environments, and visual integration with third-party applications. Certain examples provide portable content enabling plug 'n play content exchange among healthcare organizations. A standardized vocabulary using common standards (e.g., LOINC, SNOMED CT, RxNorm, FDB, ICD-9, ICD-10, etc.) is used for interoperability, for example. Certain examples provide an intuitive user interface to help minimize end-user training. Certain examples facilitate user-initiated launching of third-party applications directly from a desktop interface to help provide a seamless workflow by sharing user, patient, and/or other contexts. Certain examples provide real-time (or at least substantially real time assuming some system delay) patient data from one or more information technology (IT) systems and facilitate comparison(s) against evidence-based best practices. Certain examples provide one or more dashboards for specific sets of patients. Dashboard(s) can be based on condition, role, and/or other criteria to indicate variation(s) from a desired practice, for example.

### A. Example Healthcare Information System

An information system can be defined as an arrangement of information/data, processes, and information technology that interact to collect, process, store, and provide informational output to support delivery of healthcare to one or more patients. Information technology includes computer technology (e.g., hardware and software) along with data and telecommunications technology (e.g., data, image, and/or voice network, etc.).

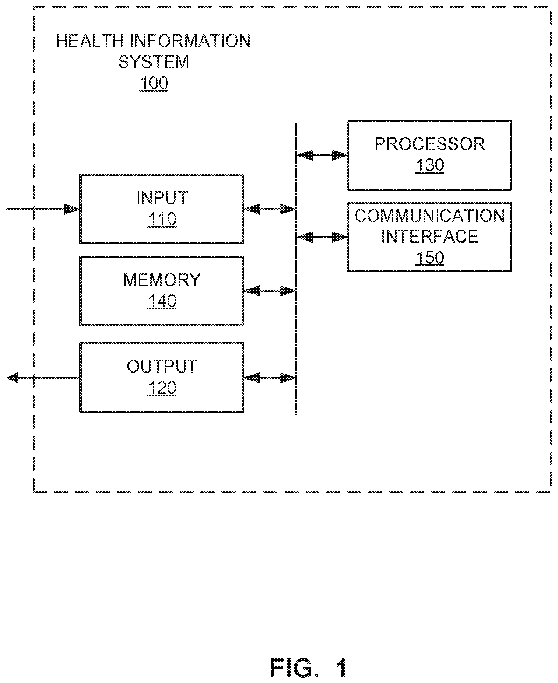

Turning now to the figures, FIG. 1 shows a block diagram of an example healthcare-focused information system 100. The example healthcare-focused information system 100 can be configured to implement a variety of systems and processes including image storage (e.g., picture archiving and communication system (PACS), etc.), image processing and/or analysis, radiology reporting and/or review (e.g., radiology information system (RIS), etc.), computerized provider order entry (CPOE) system, clinical decision support, patient monitoring, population health management (e.g., population health management system (PHMS), health information exchange (HIE), etc.), healthcare data analytics, cloud-based image sharing, electronic medical record (e.g., electronic medical record system (EMR), electronic health record system (EHR), electronic patient record (EPR), personal health record system (PHR), etc.), RTLS server, and/or other health information system (e.g., clinical information system (CIS), hospital information system (HIS), patient data management system (PDMS), laboratory information system (LIS), cardiovascular information system (CVIS), etc.

As illustrated in FIG. 1, the example healthcare-focused information system 100 includes an input 110, an output 120, a processor 130, a memory 140, and a communication interface 150. The components of the example healthcare-focused information system 100 can be integrated in one device or distributed over two or more devices.

The example input 110 of FIG. 1 may include a keyboard, a touch-screen, a mouse, a trackball, a track pad, optical barcode recognition, voice command, etc. or combination thereof used to communicate an instruction or data to the example healthcare-focused information system 100. The example input 110 may include an interface between systems, between user(s) and the healthcare-focused information system 100, etc.

The example output 120 of FIG. 1 can provide a display generated by the processor 130 for visual illustration on a monitor or the like. The display can be in the form of a network interface or graphic user interface (GUI) to exchange data, instructions, or illustrations on a computing device via the communication interface 150, for example. The example output 120 may include a monitor (e.g., liquid crystal display (LCD), plasma display, cathode ray tube (CRT), etc.), light emitting diodes (LEDs), a touch-screen, a printer, a speaker, or other conventional display device or combination thereof.

The example processor 130 of FIG. 1 includes hardware and/or software configuring the hardware to execute one or more tasks and/or implement a particular system configuration. The example processor 130 processes data received at the input 110 and generates a result that can be provided to one or more of the output 120, the memory 140, and the communication interface 150. For example, the example processor 130 can take user annotation provided via the input 110 with respect to an image displayed via the output 120 and can generate a report associated with the image based on the annotation. As another example, the example processor 130 can process updated patient information obtained via the input 110 to provide an updated patient record to an EMR via the communication interface 150.

The example memory 140 of FIG. 1 may include a relational database, an object-oriented database, a data dictionary, a clinical data repository, a data warehouse, a data mart, a vendor neutral archive, an enterprise archive, etc. The example memory 140 stores images, patient data, best practices, clinical knowledge, analytics, reports, etc. The example memory 140 can store data and/or instructions for access by the processor 130. In certain examples, the memory 140 can be accessible by an external system via the communication interface 150.

In certain examples, the memory 140 stores and controls access to encrypted information, such as patient records, encrypted update-transactions for patient medical records, including usage history, etc. In an example, medical records can be stored without using logic structures specific to medical records. In such a manner, the memory 140 is not searchable. For example, a patient's data can be encrypted with a unique patient-owned key at the source of the data. The data is then uploaded to the memory 140. The memory 140 does not process or store unencrypted data thus minimizing privacy concerns. The patient's data can be downloaded and decrypted locally with the encryption key.

For example, the memory 140 can be structured according to provider, patient, patient/provider association, and document. Provider information may include, for example, an identifier, a name, and address, a public key, and one or more security categories. Patient information may include, for example, an identifier, a password hash, and an encrypted email address. Patient/provider association information may include a provider identifier, a patient identifier, an encrypted key, and one or more override security categories. Document information may include an identifier, a patient identifier, a clinic identifier, a security category, and encrypted data, for example.

The example communication interface 150 of FIG. 1 facilitates transmission of electronic data within and/or among one or more systems. Communication via the communication interface 150 can be implemented using one or more protocols. In some examples, communication via the communication interface 150 occurs according to one or more standards (e.g., Digital Imaging and Communications in Medicine (DICOM), Health Level Seven (HL7), ANSI X12N, etc.). The example communication interface 150 can be a wired interface (e.g., a data bus, a Universal Serial Bus (USB) connection, etc.) and/or a wireless interface (e.g., radio frequency, infrared, near field communication (NFC), etc.). For example, the communication interface 150 may communicate via wired local area network (LAN), wireless LAN, wide area network (WAN), etc. using any past, present, or future communication protocol (e.g., BLUETOOTH^{™}, USB 2.0, USB 3.0, etc.).

In certain examples, a Web-based portal may be used to facilitate access to information, patient care and/or practice management, etc. Information and/or functionality available via the Web-based portal may include one or more of order entry, laboratory test results review system, patient information, clinical decision support, medication management, scheduling, electronic mail and/or messaging, medical resources, etc. In certain examples, a browser-based interface can serve as a zero footprint, zero download, and/or other universal viewer for a client device.

In certain examples, the Web-based portal serves as a central interface to access information and applications, for example. Data may be viewed through the Web-based portal or viewer, for example. Additionally, data may be manipulated and propagated using the Web-based portal, for example. Data may be generated, modified, stored and/or used and then communicated to another application or system to be modified, stored and/or used, for example, via the Web-based portal, for example.

The Web-based portal may be accessible locally (e.g., in an office) and/or remotely (e.g., via the Internet and/or other private network or connection), for example. The Web-based portal may be configured to help or guide a user in accessing data and/or functions to facilitate patient care and practice management, for example. In certain examples, the Web-based portal may be configured according to certain rules, preferences and/or functions, for example. For example, a user may customize the Web portal according to particular desires, preferences and/or requirements.

### B. Example Healthcare Infrastructure

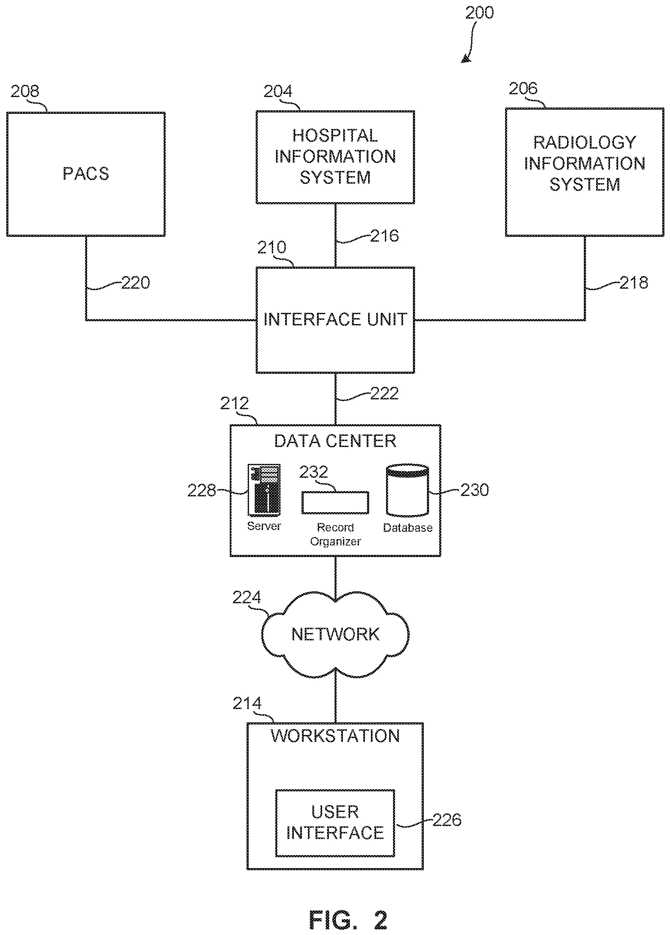

FIG. 2 shows a block diagram of an example healthcare information system (e.g., an infrastructure) 200 including one or more subsystems such as the example healthcare-related information system 100 illustrated in FIG. 1. The example healthcare information system 200 of FIG. 2 includes a HIS 204, a RIS 206, a PACS 208, an interface unit 210, a data center 212, and a workstation 214. In the illustrated example, the HIS 204, the RIS 206, and the PACS 208 are housed in a healthcare facility and locally archived. However, in other implementations, the HIS 204, the RIS 206, and/or the PACS 208 may be housed within one or more other suitable locations. In certain implementations, one or more of the HIS 204, the RIS 206, the PACS 208, etc., may be implemented remotely via a thin client and/or downloadable software solution. Furthermore, one or more components of the healthcare information system 200 can be combined and/or implemented together. For example, the RIS 206 and/or the PACS 208 can be integrated with the HIS 204, the PACS 208 can be integrated with the RIS 206, and/or the three example information systems 204, 206, and/or 208 can be integrated together. In other example implementations, the healthcare information system 200 includes a subset of the illustrated information systems 204, 206, and/or 208. For example, the healthcare information system 200 may include only one or two of the HIS 204, the RIS 206, and/or the PACS 208. Information (e.g., scheduling, test results, exam image data, observations, diagnosis, etc.) can be entered into the HIS 204, the RIS 206, and/or the PACS 208 by healthcare practitioners (e.g., radiologists, physicians, and/or technicians) and/or administrators before and/or after patient examination. One or more of the HIS 204, the RIS 206, and/or the PACS 208 can include and/or communicate with an RTLS server and can communicate with equipment and system(s) in an operating room, patient room, etc., to track activity, correlate information, generate reports and/or next actions, and the like.

In the illustrated example of FIG. 2, the HIS 204 stores medical information such as clinical reports, patient information, and/or administrative information received from, for example, personnel at a hospital, clinic, and/or a physician's office (e.g., an EMR, EHR, PHR, etc.). The example RIS 206 of the illustrated example of FIG. 2 stores information such as, for example, radiology reports, radiology exam image data, messages, warnings, alerts, patient scheduling information, patient demographic data, patient tracking information, and/or physician and patient status monitors. Additionally, the RIS 206 enables exam order entry (e.g., ordering an x-ray of a patient) and image and film tracking (e.g., tracking identities of one or more people that have checked out a film). In some examples, information in the RIS 206 is formatted according to the HL-7 (Health Level Seven) clinical communication protocol. In certain examples, a medical exam distributor is located in the RIS 206 to facilitate distribution of radiology exams to a radiologist workload for review and management of the exam distribution by, for example, an administrator.

In the illustrated example of FIG. 2, the PACS 208 stores medical images (e.g., x-rays, scans, three-dimensional renderings, etc.) as, for example, digital images in a database or registry. In some examples, the medical images are stored in the PACS 208 using the Digital Imaging and Communications in Medicine (DICOM) format. Images are stored in the PACS 208 by healthcare practitioners (e.g., imaging technicians, physicians, radiologists) after a medical imaging of a patient and/or are automatically transmitted from medical imaging devices to the PACS 208 for storage. In some examples, the PACS 208 can also include a display device and/or viewing workstation to enable a healthcare practitioner or provider to communicate with the PACS 208.

In the illustrated example of FIG. 2, the interface unit 210 includes a HIS interface connection 216, a RIS interface connection 218, a PACS interface connection 220, and a data center interface connection 222. The example interface unit 210 facilities communication among the HIS 204, the RIS 206, the PACS 208, and/or data center 212. In the illustrated example, the interface connections 216, 218, 220, 222 are implemented by a Wide Area Network (WAN) such as a private network or the Internet. Accordingly, the interface unit 210 includes one or more communication components such as, for example, an Ethernet device, an asynchronous transfer mode (ATM) device, an 802.11 device, a DSL modem, a cable modem, a cellular modem, etc. In turn, the data center 212 communicates with the workstation 214, via a network 224, implemented at a plurality of locations (e.g., a hospital, clinic, doctor's office, other medical office, or terminal, etc.). The network 224 is implemented by, for example, the Internet, an intranet, a private network, a wired or wireless Local Area Network, and/or a wired or wireless Wide Area Network. In some examples, the interface unit 210 also includes a broker (e.g., a Mitra Imaging's PACS Broker) to allow medical information and medical images to be transmitted together and stored together.

In the illustrated example, the interface unit 210 receives images, medical reports, administrative information, exam workload distribution information, and/or other clinical information from the information systems 204, 206, 208 via the corresponding interface connections 216, 218, 220. If necessary (e.g., when different formats of the received information are incompatible), the interface unit 210 translates or reformats (e.g., into Structured Query Language ("SQL") or standard text) the medical information, such as medical reports, to be properly stored at the data center 212. The reformatted medical information can be transmitted using a transmission protocol to enable different medical information to share common identification elements, such as a patient name or social security number. Next, the interface unit 210 transmits the medical information to the data center 212 via the data center interface connection 222. Finally, medical information is stored in the data center 212 in, for example, the DICOM format, which enables medical images and corresponding medical information to be transmitted and stored together.

The medical information is later viewable and easily retrievable at the workstation 214 (e.g., by their common identification element, such as a patient name or record number). The workstation 214 can be any equipment (e.g., a personal computer) capable of executing software that permits electronic data (e.g., medical reports) and/or electronic medical images (e.g., x-rays, ultrasounds, MRI scans, etc.) to be acquired, stored, or transmitted for viewing and operation. The example workstation 214 of FIG. 2 receives commands and/or other input from a user via, for example, a keyboard, mouse, track ball, microphone, etc. The workstation 214 is capable of implementing a user interface 226 to enable a healthcare practitioner and/or administrator to interact with the healthcare information system 200. For example, in response to a request from a physician, the user interface 226 presents a patient medical history. In other examples, a radiologist is able to retrieve and manage a workload of exams distributed for review to the radiologist via the user interface 226. In further examples, an administrator reviews radiologist workloads, exam allocation, and/or operational statistics associated with the distribution of exams via the user interface 226. In some examples, the administrator adjusts one or more settings or outcomes via the user interface 226.

The example data center 212 of FIG. 2 is an archive to store information such as images, data, medical reports, and/or, more generally, patient medical records. In addition, the data center 212 can also serve as a central conduit to information located at other sources such as, for example, local archives, hospital information systems/radiology information systems (e.g., the HIS 204 and/or the RIS 206), or medical imaging/storage systems (e.g., the PACS 208 and/or connected imaging modalities). That is, the data center 212 can store links or indicators (e.g., identification numbers, patient names, or record numbers) to information. In the illustrated example, the data center 212 is managed by an application server provider (ASP) and is located in a centralized location that can be accessed by a plurality of systems and facilities (e.g., hospitals, clinics, doctor's offices, other medical offices, and/or terminals). In some examples, the data center 212 can be spatially distant from the HIS 204, the RIS 206, and/or the PACS 208.

In the illustrated example, the example data center 212 of FIG. 2 includes a server 228, a database 230, and a record organizer 232. The server 228 receives, processes, and conveys information to and from the components of the healthcare information system 200. The database 230 stores the medical information described herein and provides access thereto. The example record organizer 232 of FIG. 2 manages patient medical histories, for example. The record organizer 232 can also assist in procedure scheduling, for example.

Certain examples can be implemented as cloud-based clinical information systems and associated methods of use. An example cloud-based clinical information system enables healthcare entities (e.g., patients, clinicians, sites, groups, communities, and/or other entities) to share information via web-based applications, cloud storage and cloud services. For example, the cloud-based clinical information system may enable a first clinician to securely upload information into the cloud-based clinical information system to allow a second clinician to view and/or download the information via a web application. Thus, for example, the first clinician may upload an x-ray image into the cloud-based clinical information system, and the second clinician may view the x-ray image via a web browser and/or download the x-ray image onto a local information system employed by the second clinician.

In certain examples, users (e.g., a patient and/or care provider) can access functionality provided by the healthcare information system 200 via a software-as-a-service (SaaS) implementation over a cloud or other computer network, for example. In certain examples, all or part of the healthcare information system 200 can also be provided via platform as a service (PaaS), infrastructure as a service (IaaS), etc. For example, the healthcare information system 200 can be implemented as a cloud-delivered Mobile Computing Integration Platform as a Service. A set of consumer-facing Web-based, mobile, and/or other applications enable users to interact with the PaaS, for example.

### C. Industrial Internet Examples

The Internet of things (also referred to as the "Industrial Internet") relates to an interconnection between a device that can use an Internet connection to talk (e.g., communicate) with other devices on the network. Using the connection, devices can communicate to trigger events/actions (e.g., changing temperature, turning on/off, providing a status, etc.). In certain examples, machines can be merged with "big data" to improve efficiency and operations, providing improved data mining, facilitate better operation, etc.

Big data can refer to a collection of data so large and complex that it becomes difficult to process using traditional data processing tools/methods. Challenges associated with a large data set include data capture, sorting, storage, search, transfer, analysis, and visualization. A trend toward larger data sets is due at least in part to additional information derivable from analysis of a single large set of data, rather than analysis of a plurality of separate, smaller data sets. By analyzing a single large data set, correlations can be found in the data, and data quality can be evaluated.

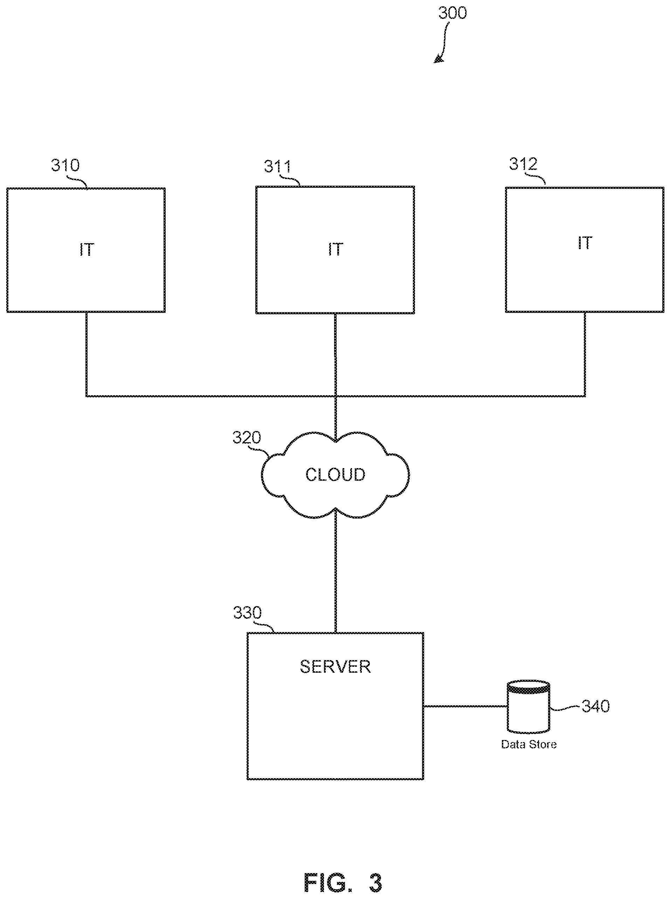

FIG. 3 illustrates an example industrial internet configuration 300. The example industrial internet configuration 300 includes a plurality of health-related assets 310-312 (sometimes referred to herein as health-focused systems or infrastructures) (e.g., information systems, imaging modalities, etc.), such as a plurality of health information systems 100 (e.g., PACS, RIS, EMR, etc.) communicating via the industrial internet configuration 300. The example industrial internet configuration 300 of FIG. 3 includes a plurality of health-related assets 310-312 communicating with a server 330 and an associated data store 340 via a cloud 320.

As shown in the example of FIG. 3, a plurality of health-related assets 310-312 can access the cloud 320, which connects the assets 310-312 with the server 330 and the associated data store 340. Information systems, for example, include communication interfaces to exchange information with the server 330 and the data store 340 via the cloud 320. Other assets, such as medical imaging scanners, patient monitors, etc., can be outfitted with sensors and communication interfaces to enable them to communicate with each other and with the server 330 via the cloud 320.

Thus, the example health-related assets 310-312 within the industrial internet configuration 300 become "intelligent" as a network with advanced sensors, controls, analytical-based decision support and hosting software applications. Using such an infrastructure, advanced analytics can be provided to associated data. The analytics combines physics-based analytics, predictive algorithms, automation, and deep domain expertise. Via the example cloud 320, the health-related assets 310-312 and associated people can be connected to support more intelligent design, operations, maintenance, and higher server quality and safety, for example.

Using the industrial internet infrastructure, for example, a proprietary machine data stream can be extracted from the asset 310. Machine-based algorithms and data analysis are applied to the extracted data. Data visualization can be remote, centralized, etc. Data is then shared with authorized users, and any gathered and/or gleaned intelligence is fed back into the assets 310-312.

### D. Data Mining Examples

Imaging informatics includes determining how to tag and index a large amount of data acquired in diagnostic imaging in a logical, structured, and machine-readable format. By structuring data logically, information can be discovered and utilized by algorithms that represent clinical pathways and decision support systems. Data mining can be used to help ensure patient safety, reduce disparity in treatment, provide clinical decision support, etc. Mining both structured and unstructured data from radiology reports, as well as actual image pixel data, can be used to tag and index both imaging reports and the associated images themselves.

### E. Example Methods of Use

Clinical workflows are typically defined to include one or more steps or actions to be taken in response to one or more events and/or according to a schedule. Events may include receiving a healthcare message associated with one or more aspects of a clinical record, opening a record(s) for new patient(s), receiving a transferred patient, reviewing and reporting on an image, and/or any other instance and/or situation that requires or dictates responsive action or processing. The actions or steps of a clinical workflow may include placing an order for one or more clinical tests, scheduling a procedure, requesting certain information to supplement a received healthcare record, retrieving additional information associated with a patient, providing instructions to a patient and/or a healthcare practitioner associated with the treatment of the patient, radiology image reading, and/or any other action useful in processing healthcare information. The defined clinical workflows may include manual actions or steps to be taken by, for example, an administrator or practitioner, electronic actions or steps to be taken by a system or device, and/or a combination of manual and electronic action(s) or step(s). While one entity of a healthcare enterprise may define a clinical workflow for a certain event in a first manner, a second entity of the healthcare enterprise may define a clinical workflow of that event in a second, different manner. In some examples, different healthcare entities may treat or respond to the same event or circumstance in different fashions. Differences in workflow approaches may arise from varying preferences, capabilities, requirements or obligations, standards, protocols, etc. among the different healthcare entities.

In certain examples, a medical exam conducted on a patient can involve review by a healthcare practitioner, such as a radiologist, to obtain, for example, diagnostic information from the exam. In a hospital setting, medical exams can be ordered for a plurality of patients, all of which require review by an examining practitioner. Each exam has associated attributes, such as a modality, a part of the human body under exam, and/or an exam priority level related to a patient criticality level. Hospital administrators, in managing distribution of exams for review by practitioners, can consider the exam attributes as well as staff availability, staff credentials, and/or institutional factors such as service level agreements and/or overhead costs.

Additional workflows can be facilitated such as bill processing, revenue cycle management, population health management, patient identity, consent management, etc.

### III. Example Hospital Tracking Network

The foregoing systems and methods can be deployed to provide real-time location services. Real-time location services (RTLS) facilitate tracking people and assets in an industrial setting, such as a hospital. The example RTLS system described herein is designed to create location awareness of assets by capturing location and proximity information from beacon tags installed throughout the hospital. Examples disclosed herein utilize reader badges worn by healthcare workers (e.g., doctors, nurses, administrators, janitors, etc.) that receive beacon messages from beacon tags that are installed in and/or affixed to assets such as hallways, rooms, equipment, patients, etc. for which location and/or proximity information is to be collected between the beacon tags and the tagged asset. For example, the beacon tags may broadcast beacon messages including a unique identifier (e.g., a signature, a MAC address, a serial number, etc.) associated with the corresponding beacon tags. As the healthcare workers walk around the hospital, their reader badges collect beacon messages transmitted from beacon tags throughout the hospital. In some disclosed examples, the reader badges aggregate the beacon messages and transmit a batch of beacon messages to an RTLS server for processing. The example RTLS server disclosed herein processes the beacon messages to create location awareness through proximity and probability.

In some disclosed examples, beacon tags are installed in and/or attached to fixed-location (e.g., placed on stationary (or near stationary)) assets. For example, some "known location" beacon tags may be affixed to hallways, doors, windows, sinks, etc. As disclosed below, in some examples, the RTLS server utilizes the beacon messages received from "known location" beacon tags to determine a location for the reader badge.

In some disclosed examples, beacon tags are affixed to mobile assets such as equipment. For example, some "mobile location" beacon tags may be affixed to beds, wheelchairs, patients, etc. As disclosed below, in some examples, the RTLS server utilizes the beacon messages received from the "mobile location" beacon tags to determine what assets are near the corresponding reader badges (e.g., the reader badge that aggregated and transmitted a batch of beacon messages).

In addition, comparing the asset locations during different timestamp intervals may be useful in determining how the assets were moved and/or when caregivers interacted with the assets. For example, consider an example in which a wheelchair (e.g., a mobile-location asset) is located in a first patient room. In the illustrated example, assume that the wheelchair is affixed with a mobile-location asset beacon tag and that the first patient room is affixed with a fixed-location asset beacon tag. In the illustrated example, when a caregiver wearing a reader badge walks into the first patient room, their reader badge collects beacon messages broadcast by the wheelchair beacon tag and the first patient room beacon tag. In the illustrated example, the caregiver location is assigned to the first patient room based on the beacon messages broadcast by the first patient room beacon tag. In addition, since the wheelchair is "seen" in the same location, the wheelchair location may also be updated to the first patient room.

In the illustrated example, while the caregiver is in the first patient room, their reader badge collects beacon messages broadcast by the wheelchair beacon tag and the first patient room beacon tag. If the caregiver begins moving the wheelchair (e.g., from the first patient room to a second patient room), their reader badge will continue to collect beacon tags broadcast by the first patient room badge tag, but will also begin collecting beacon messages broadcast by a second patient room beacon tag. In the illustrated example, once the caregiver enters the second patient room, the caregiver location is updated to the second patient room. Additionally, in the illustrated example, since the wheelchair is still "seen" by the caregiver (e.g., the wheelchair location is determined to be proximate to the caregiver), the location of the wheelchair is also updated to the second patient room.

In the illustrated example, after the wheelchair is moved from the first patient room to the second patient room, confidence that the wheelchair is located in the second patient room rather than the first patient room may be low. However, in the illustrated example, each time a caregiver walks into the first patient room and does not "see" the wheelchair, confidence that the wheelchair is located in the first patient room decreases. Additionally, in the illustrated example, each time a caregiver walks into the second patient room and does "see" the wheelchair, confidence that the wheelchair is located in the second patient room increases. In the illustrated example, the "crowd" (e.g., the caregivers) provides different snapshots of what is "seen" at different locations and at different times. As disclosed herein, an RTLS server may analyze the different snapshots to facilitate proximity detection and location tracking of assets in an environment.

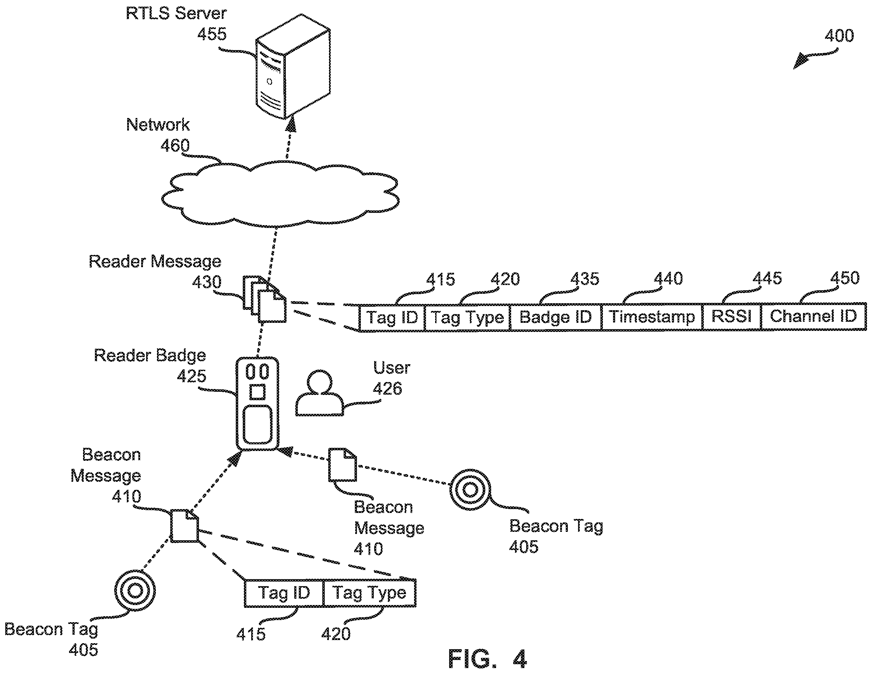

Referring to FIG. 4, an example environment 400 in which examples disclosed herein may be implemented to facilitate proximity detection and location tracking using a mobile wireless bridge is illustrated. The example environment 400 of FIG. 4 includes example beacon tags 405, an example reader badge 425 and an example real-time locations services (RTLS) server 455.

In the illustrated example of FIG. 4, the beacon tags 405 are implemented using low-power BLE transmitters and include a single coin-cell battery. In some examples, the single coin-cell battery provides power to the corresponding beacon tag 405 for two or more years. In the illustrated example, beacon tags 405 are installed throughout the environment 400 on two types of assets. For example, one or more beacon tag(s) 405 may be located on (e.g., affixed to) fixed-location assets such as doors, rooms, hallways, water fountains, etc. In addition, one or more beacon tag(s) 405 may be located on (e.g., affixed to) mobile-location assets such as patients (e.g., inserted within a patient tag), beds, IV pumps, wheelchairs, etc. Although the illustrated example of FIG. 4 includes only two beacon tags 405, other environments are likely to include additional beacon tags. For example, different environments may include tens, hundreds and/or thousands of beacon tags affixed to assets. In general, accuracy of the proximity detection and location tracking of assets in an environment is increased and/or decreased based on adding or reducing the number of beacon tags placed in the environment.

In the illustrated example of FIG. 4, the example beacon tags 405 periodically advertise their presence in the environment 400. For example, the beacon tags 405 may broadcast example beacon messages 410 every one second. In other examples, the beacon tags 405 may broadcast beacon messages 410 aperiodically and/or as a one-time event. In some examples, the beacon tags 405 may broadcast beacon messages 410 at different time intervals. For example, beacon tags 405 located on fixed-location assets may broadcast beacon messages 410 every two seconds, while beacon tags 405 located on mobile-location assets may broadcast beacon messages 410 every second. In some examples, beacon tags located on mobile-locations assets may broadcast beacon messages 410 at a first frequency (e.g., once every second) while the mobile-location asset is stationary and may broadcast beacon messages 410 at a second frequency (e.g., once every half-second) while the mobile-location asset is moving. However, other time intervals may additionally or alternatively be used.

In the illustrated example, the beacon messages 410 include tag identifying information 415 and tag-type identifying information 420. For example, tag identifying information 415 may be a unique identifier of the beacon tag 405 such as a MAC address, a serial number, an alphanumeric signature, etc. The example tag-type identifying information 420 identifies whether the beacon tag 405 broadcasting the beacon message 410 is affixed to a fixed-location asset or affixed to a mobile-location asset. However, the beacon messages 410 may include additional or alternative information. For example, the beacon messages 410 may include information identifying the software version being executed by the beacon tags 405, may include information identifying a power level of the beacon tag 405, etc.

In the illustrated example of FIG. 4, the beacon messages 410 are received by the reader badge 425. In the illustrated example, the reader badge 425 is worn by a hospital caregiver 426 such as a doctor, a nurse, etc. As the hospital caregiver moves through the hospital, the reader badge 425 collects beacon messages 410 broadcast by the beacon tags 405. For example, while the hospital worker 426 is visiting a patient in an example patient room #1, the example reader badge 410 may collect one or more beacon message(s) from a fixed-location asset beacon tag located on a door of the patient room #1, one or more beacon message(s) from a fixed-location asset beacon tag located on a sink in the patient room #1, one or more beacon message(s) from a mobile-location asset beacon tag located on the patient's identification tag, one or more beacon message(s) from a mobile-location asset beacon tag located on a bed in the patient room #1, etc.

In the illustrated example of FIG. 4, the reader badge 425 generates example reader messages 430 in response to receiving the beacon messages 410. For example, the reader badge 425 may create a reader message 430 including the tag identifying information 415 and the tag-type identifying information 420 included in the beacon message 410 and append example badge identifying information 435, an example timestamp 440, example signal strength information 445, and example channel identifying information 450. In the illustrated example, the badge identifying information 435 is a string of alphanumeric characters that uniquely identifies the reader badge 410 (e.g., a MAC address, a serial number, an alphanumeric signature, etc.). The example timestamp 440 identifies a date and/or time (e.g., January 1, 2015, 9:10:04 pm) when the beacon message 410 was received by the reader badge 425. The example signal strength information 445 identifies signal strength of the beacon message 410 when it was received by the reader badge 425 (e.g., a received signal strength indication (RSSI) value). The example channel identifying information 450 identifies a channel on which the beacon message 410 was received (e.g., a Bluetooth frequency channel such as channel 37, channel 38 or channel 39).

In the illustrated example of FIG. 4, the reader badge 425 periodically communicates a group (e.g., a batch) of reader messages 430 to the RTLS server 455. For example, the reader badge 425 may transmit one or more reader messages 430 that were collected over a period of time (e.g., thirty seconds). Additionally or alternatively, the reader badge 425 may communicate one or more reader message(s) 430 aperiodically and/or as a one-time event. For example, the reader badge 425 may collect a threshold number of reader messages 430 prior to transmitting the collected reader messages 430 to the RTLS server 455. In some examples, the reader badge 425 transmits the reader messages 430 as they are created by the reader badge 425.

In the illustrated example of FIG. 4, the RTLS server 455 is a server and/or database that facilitates proximity detection and location tracking. In some examples, the RTLS server 455 is implemented using multiple devices. For example, the RTLS server 455 may include disk arrays or multiple workstations (e.g., desktop computers, workstation servers, laptops, etc.) in communication with one another.

In the illustrated example, the RTLS server 455 is in communication with the reader badge 425 via one or more wireless networks represented by example network 460. Example network 460 may be implemented using any suitable wireless network(s) including, for example, one or more data busses, one or more wireless Local Area Networks (LANs), one or more cellular networks, the Internet, etc. As used herein, the phrase "in communication," including variances thereof (e.g., communicates, in communication with, etc.), encompasses direct communication and/or indirect communication through one or more intermediary components and does not require direct physical (e.g., wired) communication and/or constant communication, but rather additionally includes communication at periodic or aperiodic intervals, as well as one-time events.

In the illustrated example of FIG. 4, the RTLS server 455 utilizes the reader messages 430 to facilitate proximity detection and location tracking of assets in the environment 400. In the illustrated example, the RTLS server 455 selects a portion of reader messages 430 received from the reader badge 425 to determine a location of the reader badge 425. For example, the RTLS server 455 may process the reader messages 430 to identify a first subset of reader messages 430 (e.g., one or more reader messages) that were received by the reader badge 425 during a first window of interest (e.g., a five second window) and that were fixed-location asset tag type (e.g., based on the tag-type information 420 included in the first subset of reader messages). In the illustrated example of FIG. 4, the RTLS server 455 utilizes the signal strength information 445 included in the first subset of reader messages 430 to determine a nearest fixed-location asset. For example, a relatively stronger RSSI value may indicate that the broadcasting beacon tag 405 is closer in proximity to the reader badge 425 than a beacon tag 405 associated with a relatively weaker RSSI value. In the illustrated example of FIG. 4, the RTLS server 455 updates the location of the reader badge 425 based on the nearest fixed-location asset.

In the illustrated example of FIG. 4, once the RTLS server 455 associates the reader badge 425 with a location (e.g., the location of the nearest fixed-location asset), the RTLS server 455 identifies a second subset of reader messages 430 (e.g., one or more reader messages) that were received by the reader badge 425 during the first window of interest (e.g., a five second window) and that were mobile-location asset tag type (e.g., based on the tag-type information 420 included in the second subset of reader messages 430). For example, the RTLS server 455 may update the location of a mobile-location asset based on its proximity to the reader badge 425.

In the illustrated example of FIG. 4, the RTLS server 455 selects a reader message of the second subset of reader messages 430 and classifies the corresponding mobile-location assets relative location to the reader badge 425 based on the RSSI value 455 included in the selected reader badge 430. For example, the RTLS server 455 classifies mobile-location asset as relatively-far assets when the signal strength information 455 satisfies a first threshold (e.g., the RSSI value is less than (-60) decibels). The example RTLS server 455 of FIG. 4 classifies mobile-location assets as relatively-immediate assets when the signal strength information 455 satisfies a second threshold (e.g., the RSSI value is greater than (-40) decibels). In the illustrated example of FIG. 4, the RTLS server 455 classifies mobile-location assets as relatively-near assets when the signal strength information 455 does not satisfy the first threshold and the second threshold. For example, the RTLS server 455 may classify mobile-location assets as relatively-near assets when the RSSI value is less than (-40) decibels and greater than (-60) decibels.

In the illustrated of FIG. 4, depending on the relative location classifications, the RTLS server 455 updates the location of the mobile-location asset and/or updates an asset-location confidence score associated with the mobile-location asset. In the illustrated example, the asset-location confidence score represents a probability (or likelihood) that a mobile-location asset may be found at the currently assigned asset-location. For example, when a mobile-location asset is "seen" in the same location, the RTLS server 455 increases the asset-location confidence score of the mobile-location asset. When the mobile-location asset is "seen" in a different location, the RTLS server 455 decreases the asset-location confidence score of the mobile-location asset. Additionally, when the asset-location confidence score fails to satisfy a location threshold (e.g., is less than a location threshold), the asset-location of the mobile-location asset may be updated based on, for example, the location of the reader badge 425 that collected the beacon message 410 emitted from the mobile-location asset (e.g., by the beacon tag 405 affixed to the mobile-location asset).

In the illustrated example, when a mobile-location asset is classified as relatively-far, the example RTLS server 455 of FIG. 4 discards the reader message 430 and the RTLS server 455 makes not change to the location of the mobile-location asset and/or the asset-location confidence score associated with the mobile-location asset. For example, the reader badge 425 may have collected a relatively weak beacon message emitted from a mobile-location asset passing through the hallway outside of the patient room #1. In some examples, the reader badge 425 may filter such beacon messages (e.g., beacon messages 410 that are associated with weak (e.g., low) RSSI values) rather than communicate the weak beacon messages to the RTLS server 455.

When a mobile-location asset is classified as a relatively-immediate asset, high signal strength (e.g., an RSSI value greater than (-40) decibels) may be indicative of a mobile-location asset that is in-front of the hospital worker 426, is being used by the hospital worker 426 and/or is being moved by the hospital worker 426. In some such instances, the location of the mobile-location asset may be assumed to be the same as the location of the reader badge 425. In the illustrated example, the example RTLS server 455 of FIG. 4 updates the location of the mobile-location asset to the location of the reader badge 425. In addition, the example RTLS server 455 increments the asset-location confidence score of the mobile-location asset (e.g., the probability of the mobile-location asset being located at the updated asset-location is increased). In some examples, if the beacon tag 405 is relatively-immediate to the reader badge 425, an assumption may be made that the caregiver is interacting with the corresponding assets. For example, the caregiver may be pushing a patient in a wheelchair.

In the illustrated example of FIG. 4, when a mobile-location asset is classified as a relatively-near asset (e.g., is associated with a medium signal strength), the example RTLS server 455 of FIG. 4 compares the current location associated with the mobile-location asset to the location of the reader badge 425. In the illustrated example, the RTLS server 455 increases the asset-location confidence score of the mobile-location asset when the current asset-location is the same as the location of the reader badge 425. For example, the mobile-location asset is "seen" in the same location as it is currently assigned. In some examples when the current asset-location is not the same as the location of the reader badge 425, the example RTLS server 455 decreases the asset-location confidence score of the mobile-location asset. In addition, the example RTLS server 455 compares the asset-location confidence score of the mobile-location asset to a location threshold and, when the asset-location confidence score fails to satisfy the location threshold (e.g., is less than the location threshold), the RTLS server 455 updates the asset-location of the mobile-location asset to the location of the reader badge 425 that received the corresponding beacon message 410.

In the illustrated example of FIG. 4, the example environment 400 includes an example dock module 465. The example dock module 465 may be used to charge one or more reader badges 425. In some examples, the dock module 465 receives beacon messages 410 from beacon tags 405 and/or transmits reader messages 430 to the RTLS server 455.

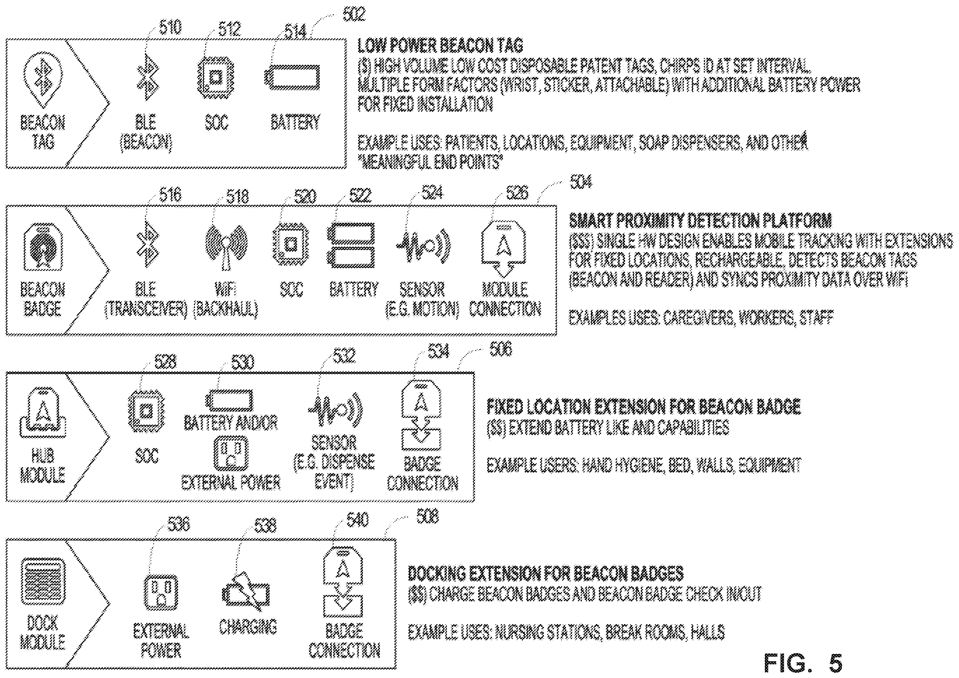

FIG. 5 illustrates various components included in an example beacon tag 502, an example beacon badge 504, an example hub module 506 and example dock module 508. For example, the beacon tag 502 includes one or more BLE chips (labeled "Beacon") 510 to transmit beacon messages 410, one or more power sources 514 (e.g., one or more coin-cell batteries) and a system-on-a-chip (SOC) 512 to manage the one or more BLE chips 510 and the one or more power sources 514. The example beacon badge 504 includes one or more BLE chips 516 (labeled "transceiver") to receive beacon messages 406a -409a, one or more Wi-Fi chips 518 to communicate with a wireless network (e.g., the example network 460), one or more power sources (e.g., one or more batteries) 522, one or more sensors 524 (e.g., a motion sensor, an accelerometer, a gyroscope, etc.) and a system-on-a-chip (SOC) 520 to manage the one or more BLE chips 516, the one or more Wi-Fi chips 518, the one or more power sources 522 and the one or more sensors 524. The example beacon badge 504 also includes an example module connector 526 to connect the beacon badge 504 to the example hub module 506 and/or the dock module 508.

In the illustrated example of FIG. 5, the beacon badge 504 is connectable to the example hub module 506. The connection between the beacon badge 504 and the hub module 506 may include a mechanical connection, an electrical connection, or combinations thereof. In the illustrated example, the hub module 506 may be used to track asset interactions with fixed locations. In a healthcare environment, examples of fixed locations include soap dispensers, beds, walls, equipment, etc. In other environments, such as a retail environment, fixed locations may include wall sconces, light fixtures, mirrors, shelving, and other such fixed locations.

The hub module 506 may be leveraged to identify particular locations. As an example, the beacon badge 504 may be coupled, via a badge connection 534, to a hub module 506 placed on an entrance to a restricted area to identify when a person wearing a beacon tag 502 enters (or approaches) the restricted area. In one embodiment, the hub module 506 includes a system-on-a-chip (SOC) 528 to manage components of the hub module 506, one or more power sources 530 (e.g., one or more batteries and an external power source (e.g., an AC/DC connection)) to extend the battery life and capabilities of the beacon badge 504, one or more sensors 532 communicatively coupled to the SOC 528, and a badge connection 534 for connecting the beacon badge 504 to the hub module 506.

In the illustrated example, the beacon badge 504 may be connectable (e.g., mechanically coupled, electronically coupled, etc.) to the example dock module 508. In the illustrated example, the dock module 508 may be used to charge one or more beacon badges 504. Accordingly, and in one embodiment, the dock module 508 includes an external power connector 536 (e.g., an AC connector), a charging indicator 538 to indicate whether the beacon badge 504 is charged or charging, and a badge connection 540 for connecting the beacon badge 504 to the dock module 508. In one embodiment, the dock module 508 is portable. For example, the dock module 508 may be placed throughout one or more environments, such as at cash registers, podiums, counters, nursing stations, break rooms, hallways, etc., and a caregiver may couple their beacon badge 504 to the dock module 508, via a badge connection 540, when they are off-duty.

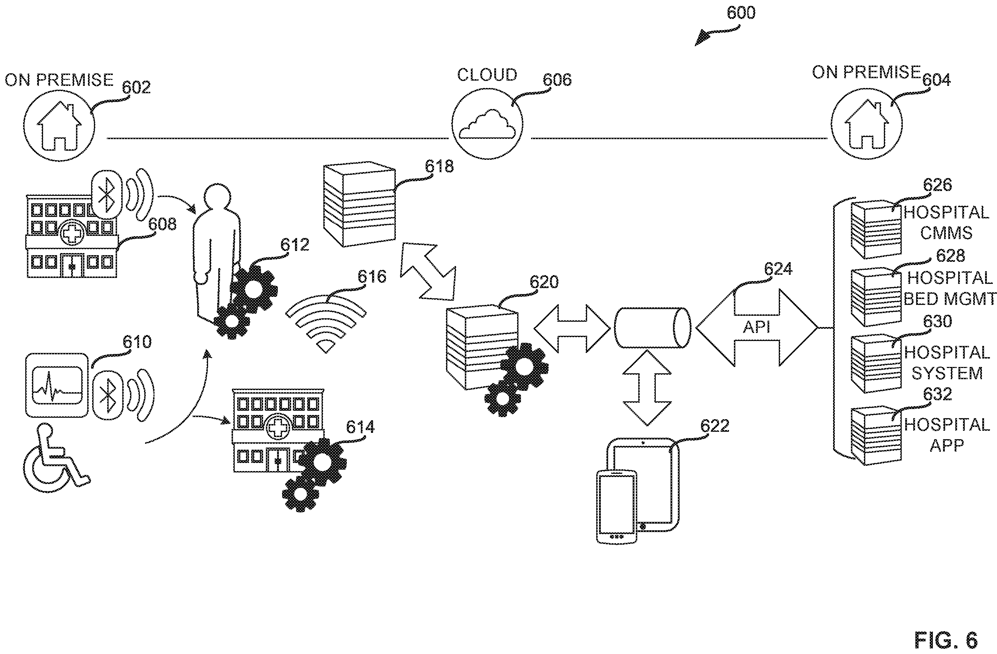

FIG. 6 illustrates an example environment 600 illustrating interaction between premises 602, 604 via a cloud 606. In the example of FIG. 6, one or more fixed beacons 608 and one or more mobile beacons 610 are positioned in a facility 602 (e.g., a hospital, clinic, etc.). The beacons 608, 610 are affixed (e.g., permanently affixed, removably affixed, etc.) to locations, assets, etc. For example, the fixed beacon 608 can be mounted on a wall at a location in the facility 602 at which asset(s) may be located to provide a location to a receiver. The mobile beacon 610 can be affixed (e.g., permanently, removably, etc.) to an item to be located and tracked (e.g., an intravenous (IV) pump, imaging scanner (e.g., x-ray, CT, ultrasound, etc.), crash cart, lab cart, etc.), for example.

The beacons 608, 610 are detected and read (e.g., via Bluetooth^{™}, Bluetooth Low Energy (BLE), near field communication (NFC), etc.) by one or more mobile receivers 612 and/or fixed receivers 614, for example. For example, the mobile receiver 612 includes logic to process its location (e.g., with respect to the fixed beacon 608, etc.). The mobile receiver 612 can be worn by a person and/or mobile asset to create a crowdsourced environment in which the mobile receiver 612 interacts with beacons 608, 610 and informs the system 600 of the receiver 612 location and presence of beacon(s) 608, 610 within range of the location, for example. The fixed receiver 614 is configured with its location in the facility 602. The fixed receiver 614 can be mounted on a wall in a location where crowdsourcing is reduced (e.g., storage locations, enclosed locations, etc.) to interact with beacons 608, 610 and inform the system 600 of the receiver 614 location and presence of beacon(s) 608, 610 within range of the location, for example. The mobile receiver(s) 612 and fixed receiver(s) 614 process which asset(s) are located within range (e.g., as indicated mobile beacon(s) 610 and/or fixed beacon(s) 608, etc.) and notify other component(s) of the system 600.

The receiver(s) 612, 614 communicate over a channel 616, such as Wi-Fi, etc., with a middleware gateway 618 to transmit information regarding beacon 608, 610 location to a middleware engine 620. The middleware gateway 618 can be an edge device, gateway device, hub, and/or other electronic device to interface between the premises 602 and the cloud 606, for example. The middleware engine 620 can reside on the cloud 606 to process received beacon 608, 610 and receiver 612, 614 data and calculate location information. The middleware engine 620 can also publish location events to one or more receiving/subscribing recipients, for example.

For example, one or more consuming applications 622 access location data from the middleware engine 620 via the cloud 606 to leverage the location data for scheduling, tracking, (re)ordering, maintenance, billing, protocol compliance, treatment evaluation, employee evaluation, resource evaluation, and/or other resource management application(s), etc. Alternatively or in addition, an application programming interface (API) 624 provides location awareness data for consumption by one or more hospital applications 626-632 at a second facility (e.g., hospital, clinic, etc.) 604. For example, a hospital computerized maintenance management system (CMMS) 626, a hospital bed management system 628, and/or other hospital system 630, hospital application 632, etc., can receive and process asset location information via the API 624.

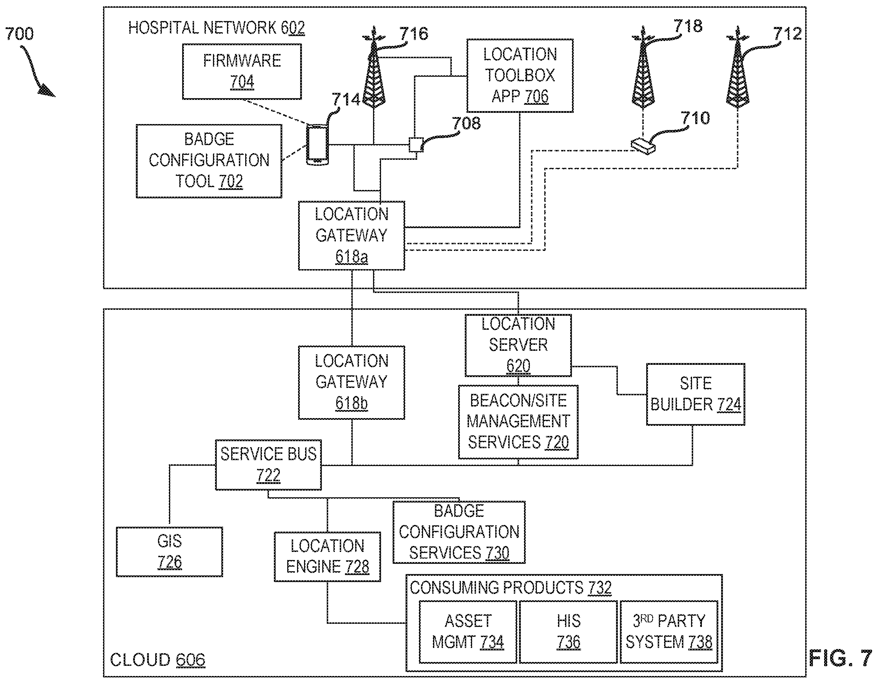

FIG. 7 illustrates an example architecture 700 of the hospital network 602 and the cloud 606 of FIG. 6. As shown in the example of FIG. 7, the hospital network 602 communicates with the cloud 606 via the middleware or location gateway 618, which can be divided (as shown in the example of FIG. 7) into a client location gateway 618a and a server location gateway 618b. The example hospital network 602 includes a badge configuration tool 702 used to configure a badge 714 (e.g., a hospital staff badge, smart phone, etc.) for one or more parameters such as WiFi network, gateway connectivity, gateway security credential/certificate, etc. The tool 702 can communicate with the badge 714 via WiFi, Bluetooth, NFC, etc. Further, the badge 714 communicates with the client location gateway 618a to provide location information to the cloud 606.

Additionally, firmware 704 can communicate with the badge 714 to update firmware, settings, etc., on the badge 714. The example firmware 704 can provide and/or be associated with a software development kit (SDK) to enable integration of application(s) into the badge 714, for example. Using the SDK, the firmware 704 can provide notifications, offers, and/or other customizations to the badge 714 and/or a user/wearer of the badge 714, for example.

The example hospital network 602 of FIG. 7 also includes a location toolbox application 706, which communicates with a beacon 716 (e.g., a Bluetooth beacon, BLE beacon, etc.) and/or a hub 708 (e.g., via Bluetooth, BLE, etc.). The beacon 716 and/or hub 708 can also communicate with the badge 714 and/or the client location gateway 618a, for example. The toolbox 706 provides configuration and/or authorization application(s), setting(s), configuration(s), etc., for the hub 708, badge 714, and/or beacon 716, etc. For example, the toolbox 706 can be used to set beaconing frequency, beacon range, beacon transmission mode, etc. The toolbox 706, beacon 716, and/or badge 714 can communicate via the hub 708 with the client location gateway 618a, etc.

The example hospital network 602 of FIG. 7 can also include a passive reader 710, access point 712, and passive tag 718. The WiFi access point 712 helps relay locating information by presence (e.g., in the facility 602), zone (e.g., in a particular area of the facility 602), location (e.g., actual location), etc. The passive tag 718 and passive reader 710 can interact to provide location information in the hospital network 602 to the client location gateway 618a, for example.

The client location gateway 618a communicates with the server location gateway 618b at the cloud 606. The client location gateway 618a also communicates with a middleware engine 620 such as a locationing server 620. The example server 620 provides a plurality of features including a management user interface (UI), a system health monitor, configuration information, insights/analytics, etc. The example server 620 communicates with beacon/site management services 720 and a site builder 724, which helps to map out a location (e.g., the hospital network 602, etc.) and beacons found at the location.

Using a service bus 722, the server location gateway 618b, beacon/site management services 720, and/or the site builder 724 can communicate with a geographic information system (GIS) 726 to create map(s) of the facility 602 to be stored using georeferenced location coordinates, for example. Fixed receivers placed in the facility 602 can be identified and added to the map using the site builder 724 and GIS 726. A location engine 728 can be used to leverage the map(s) and geographic information to associate location(s) with detected beacon events to derive a location for a particular asset, for example. Using the GIS 726 and site builder 724, maps can be modified/updated in real time (or substantially real time given some data processing, transmission, and/or storage latency, etc.) to make fast, fluid changes based on incoming data, for example. The GIS 726 provides spatial context to the inside of the facility 602 mapped by the site builder 724, for example. Using the GIS 726 platform, distance(s) between objects can be derived and georeferenced coordinates can be included. Information generated by the location engine 728 can be consumed by one or more products 732 including asset management 734, hospital information system (HIS) 736, and/or other third party system 738, etc. Badge configuration services 730 can also help with badge configuration on the server/cloud side, helping to update the badge configuration tool 702 at the hospital 602, for example.

Thus, certain examples provide systems and methods to monitor and manage badge(s), beacon(s), and receiver(s) and provide health statistics for such devices. Certain examples provide APIs that allow devices installed at a location to communicate status information to the cloud 606 infrastructure to be processed to display reports, analytics, facilitate interaction for repair/update, etc., to drive notifications, alerts, maintenance, etc., for system health and ongoing system operation. Certain examples facilitate monitoring and evaluation of network and system performance and retuning/reconfiguring/redefining desired network and/or system operation.

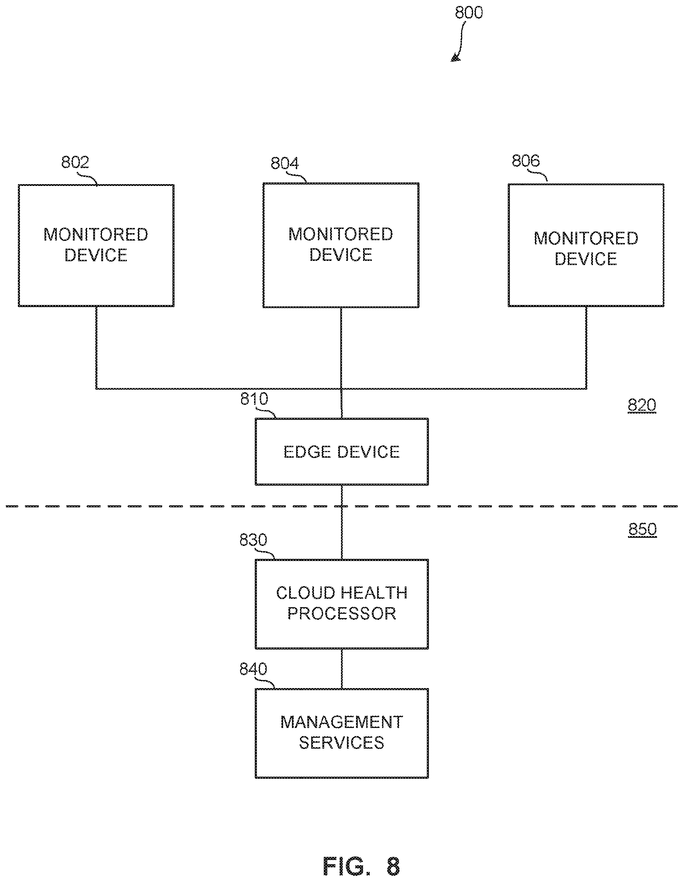

More generally, FIG. 8 illustrates a basic real time location platform 800 including a number of monitored devices 802-806 and an edge device 810 in a facility 820, along with a cloud health processor 830 and management service(s) 840 in a cloud 850. The monitored devices 802-806 can include one or more beacons, badges, and/or receivers 608, 610, 612, 614, 708, 710, 712, 714, 716, 718, etc. The edge device 810 can include the location gateway 618, etc. The cloud health processor 830 can include the location server 620, etc. The management services 840 can include beacon/site management services 720, badge configuration services 730, etc.

The cloud health processor 830 defines a mechanism and associated API specification by which location receivers 612, 614, 710 deployed as part of a real time location platform 600, 700 can transmit system health information using an event-based messaging framework. The data/events provided can be captured and utilized to maintain the system 600, 700 and help ensure optimal and/or otherwise improved performance.

In certain examples, given numerous dependencies, connectivity issues, and power concerns, the system 800 is configured such that the devices 802-806 self-report their health status and associated system events to the health processor 830 via the edge device 810 to help maintain a functional system 600, 700, 800. For example, location devices 802-806 are designed to submit event data (e.g., JSON documents, and/or other format/protocol such as XML, CSV, HTTP, JMS, SMTP, etc.) via the edge device 810 to an interface (e.g., a RESTful service interface, etc.) at the health processor 830.

A plurality of events can be defined. An event includes a set of base (e.g., header, etc.) attributes that are used for ongoing system health management. In addition, each event includes a details section in which attributes/data specific to an event type can be included. For example, numerous events can be defined, and these events can be sent in response to a specified condition (e.g., device regaining network connectivity, e.g., device placed on charger, e.g., device removed from charger, etc.) and/or on a time schedule that is configurable as part of the device profile. The following table provides some examples of receiver health-related events:

| **Event** | **Occurrence/Trigger** |
|---|---|
| On Charge | When a badge is placed on charge |
| Off Charge | When a badge is taken off charge |
| Forced Reboot | When the badge operating system restarts |
| Unforced Reboot/System Error | When the badge operating restarts after a system error |
| WiFi Reconnect | When the badge reconnects to WiFi not associated with a reboot event |
| Heartbeat | When the receiver profile configured time interval has elapsed |

Thus, each receiver transmits health/operating details to the processor 830 via the API (e.g., API 624, etc.) when associated events are executed. In certain examples, a WiFi reconnect does not include a roaming and/or access point transition for a mobile receiver. In certain examples, a heartbeat timer restarts on device reboot. In certain examples, a heartbeat interval is set to be frequent enough to monitor temperature changes, eliminating a need for temperature threshold events, for example.

In certain examples, a service interface (e.g., API) specification can be provided, such as for a RESTful service, etc., used by device(s) 802-806 to post health events. The service interface can define a health API and/or a reference API that provides definition for location events, time, firmware updates, system health, receiver configuration, etc. For example, a location event request can be formatted as a JSON object to include a beacon MAC address, UUID, RSSI, battery life (e.g., percentage of battery life remaining, battery value, etc.), timestamp (e.g., a time at which the beacon event was received, etc.), receiver MAC address, etc. A get time request can be implemented as a JSON formatted object including a time, such as a UNIX time, POSIX time, Epoch time, UTC time, etc., for example. A firmware update can be implemented as a binary file providing an application/octet stream to a target device 802-806, for example. A system health request can be implemented, for example, as a JSON formatted object including an event type, device MAC address, timestamp, firmware version, depth of discharge (e.g., percent of battery life remaining, etc.), temperature (e.g., device temperature in Fahrenheit, Celsius, etc.), details (e.g., any additional details provided for the event), etc. A receiver configuration request can be implemented, for example, as a JSON formatted object including a scan interval (e.g., a period of time for which received beacons are being evaluated to determine which beacons should be transmitted, etc.), a scan channel (e.g., BLE channel(s) on which the device should listen, etc.), heartbeat interval, WiFi transmission frequency, profile name/ID, beacon type, proximity range, RSSI low (e.g., weakest RSSI signal strength considered within the range that a beacon should be processed, etc.), RSSI high (e.g., strongest RSSI signal strength considered within the range that a beacon should be processed, etc.), beacon hit count (e.g., a number of beacon hits required to be received within a scan interval, etc.), scan retention interval (e.g., a number of scans that occur before results of a scan are stored for transmission, etc.), send closest only (e.g., if true, all beacons received within the given range will be transmitted by the device, else only the closest (e.g., highest RSSI value) beacon is to be transmitted, etc.), suppress repeats (e.g., if true, transmissions from the device will be suppressed if they are the same as the previous scan interval, etc.), time service URL (e.g., uniform resource locator exposing the time service, etc.), event service URL (e.g., uniform resource locator exposing the event service, etc.), firmware service URL (e.g., uniform resource locator exposing the firmware service, etc.), firmware filename, etc.

The quality of location data provided by the real time location platform 600, 700 is dependent on the health of the devices deployed to receive sensory/location events. If the deployed devices are not functioning as intended, the location data produced by the system has the potential to be inaccurate/unreliable. To help ensure accurate location data, support system(s) and/or team(s) (e.g., health processor 830 and management service 840) must be able to monitor system health, isolate problematic devices and correct the problems through reconfiguration, replacement, upgrade, etc. Thus, certain examples provide a centralized health and monitoring capability for large scale systems that include many thousands of devices deployed in a wide range of environments. Without this system, deployed systems would fall into disrepair over time and/or the costs of monitoring/maintaining such systems would threaten the commercial viability of the dependent products. Certain examples monitor system health and provide maintenance/solutions to enable improved system performance, higher customer satisfaction, higher return on investment for a customer, lower cost of ownership for the customer, lower support costs for a supplier, increased profit margin for the supplier, etc.

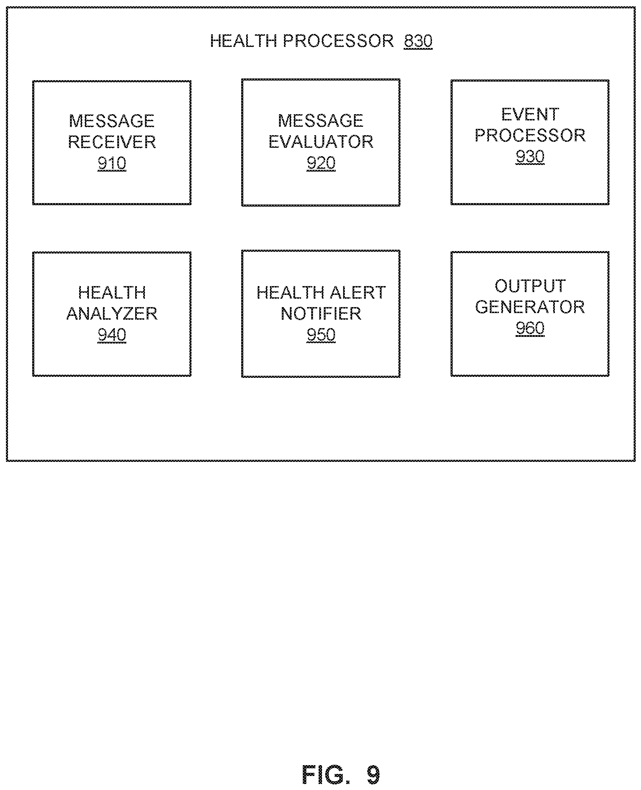

FIG. 9 illustrates an example implementation of the cloud health processor 830. In the example of FIG. 9, the processor 830 includes a message receiver 910, a message evaluator 920, an event processor 930, a health analyzer 940, a health alert notifier 950, and an output generator 960.

The example message receiver 910 monitors for a message from a receiver (e.g., from one or more devices 802-806 including one or more beacons, badges, and/or receivers 608, 610, 612, 614, 708, 710, 712, 714, 716, 718, etc.). When a message is received, the example message evaluator 920 evaluates the received message to determine a message type associated with the message (e.g., location message, firmware message, time message, receiver configuration message, health message, etc.). If the message is not a receiver health message, then, the message evaluator 920 sends the message to another processor, such as the location engine 728, site builder 724, consuming product(s) 732, etc.

If the message is a health message, then the message evaluator 920 sends the message to the event processor 930. The example event processor 930 processes the health message to identify an event type indicated by the message. For example, the message may indicate an on charge event, off charge event, forced reboot event, unforced reboot/system error event, WiFi reconnect event, heartbeat event, etc. Based on the event type, the event processor 930 processes the details of the event.

The event processor 930 provides the message details and event type to the health analyzer. Based on the event type, the example health analyzer 940 compares the details of the event to a threshold, range, standard, norm, etc. If the event is within normal or expected behavior, the event can be logged via the output generator 960. If the event is outside the prescribed bound(s), the health alert notifier 950 can be triggered in response to the event. In some examples, the health alert notifier 950 can generate a response message or instruction to the device via the output generator 960 to adjust a level, setting, mode, etc., in response to the event (e.g., not charging enough, not charging properly, irregular heartbeat, reboot needed, etc.) such as to send a message to a user, automatically adjust a device setting, trigger a maintenance request, alert hospital staff to a failing device, change in setting/configuration warranted, etc. Thus, the output generator 960 can provide an update and/or other message to the device and/or a third party (e.g., beacon/site management services 720, badge configuration services 730, consuming product(s) 732, etc.) to repair, replace, and/or adjust the affected device(s). An alert, update, and/or other message can be generated to help ensure reliable operation and uptime of the RTLS system 600, 700, for example.

While example implementations of the systems 100, 200, 300, 400, 600, 700, 800, 900 are illustrated in FIGS. 1-9, one or more of the elements, processes and/or devices illustrated in FIGS. 1-9 may be combined, divided, re-arranged, omitted, eliminated and/or implemented in any other way. Further, the example components of FIGS. 1-9 may be implemented by hardware, software, firmware and/or any combination of hardware, software and/or firmware. Thus, for example, any of the example components of FIGS. 1-9 can be implemented by one or more analog or digital circuit(s), logic circuits, programmable processor(s), application specific integrated circuit(s) (ASIC(s)), programmable logic device(s) (PLD(s)) and/or field programmable logic device(s) (FPLD(s)). When reading any of the apparatus or system claims of this patent to cover a purely software and/or firmware implementation, at least one of the example components of FIGS. 1-9 is/are hereby expressly defined to include a tangible computer readable storage device or storage disk such as a memory (e.g., a read only memory (ROM), hard drive, flash memory, other volatile and/or non-volatile memory, etc.), a digital versatile disk (DVD), a compact disk (CD), a Blu-ray disk, etc. storing the software and/or firmware. Further still, the example systems of FIGS. 1-9 may include one or more elements, processes and/or devices in addition to, or instead of, those illustrated in FIGS. 1-9, and/or may include more than one of any or all of the illustrated elements, processes and devices.

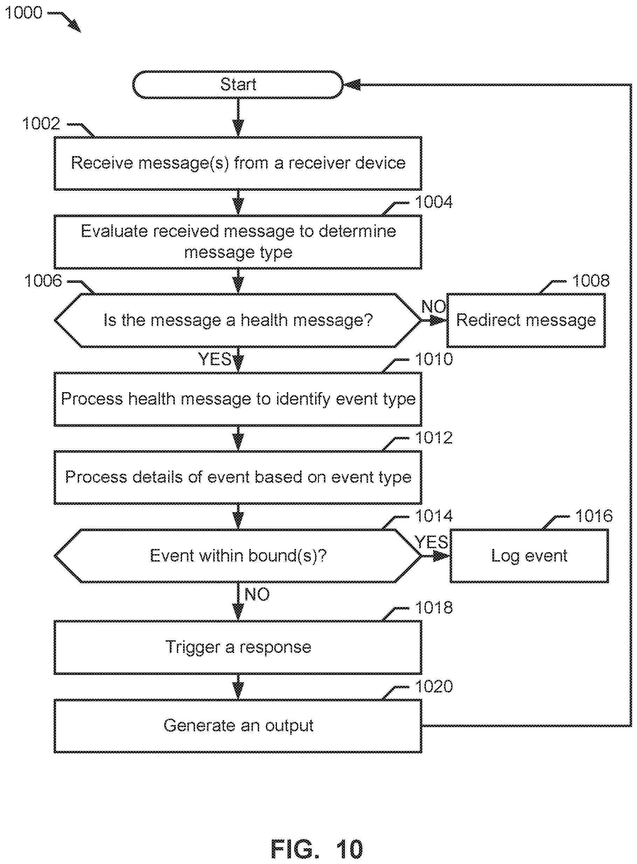

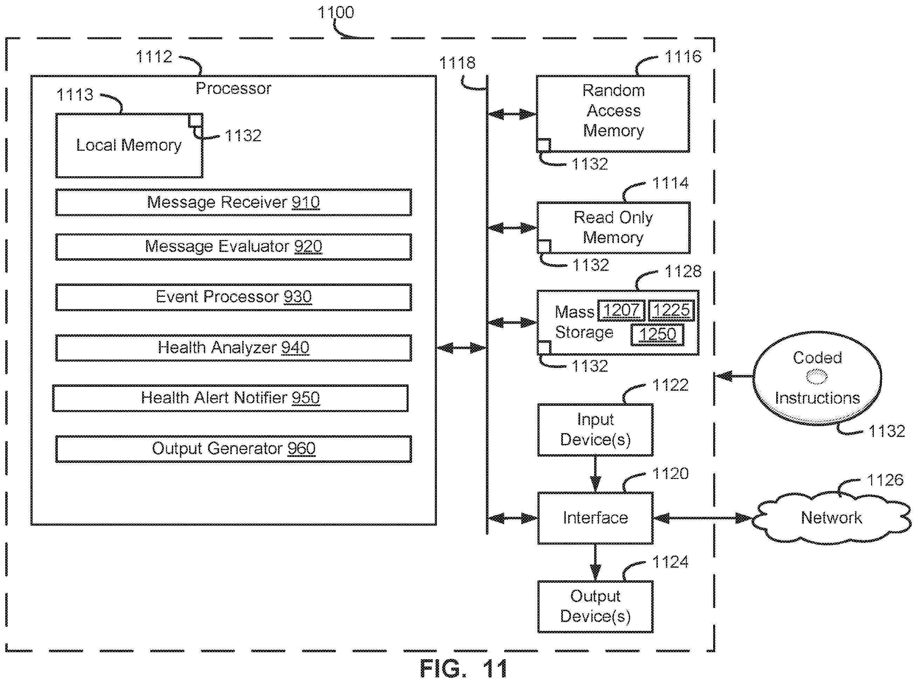

A flowchart representative of example machine readable instructions for implementing the systems of FIGS. 1-9 is shown in FIG. 10. In these examples, the machine readable instructions comprise a program for execution by a processor such as the processor 1112 shown in the example processor platform 1100 discussed below in connection with FIG. 11. The program may be embodied in software stored on a tangible computer readable storage medium such as a CD-ROM, a floppy disk, a hard drive, a DVD, a Blu-ray disk, or a memory associated with the processor 1112, but the entire program and/or parts thereof could alternatively be executed by a device other than the processor 1112 and/or embodied in firmware or dedicated hardware. Further, although the example programs are described with reference to the flowchart illustrated in FIG. 10, many other methods of implementing the example systems may alternatively be used. For example, the order of execution of the blocks may be changed, and/or some of the blocks described may be changed, eliminated, or combined.

As mentioned above, the example process(es) of FIG. 10 may be implemented using coded instructions (e.g., computer and/or machine readable instructions) stored on a tangible computer readable storage medium such as a hard disk drive, a flash memory, a ROM, a CD, a DVD, a cache, a random-access memory (RAM) and/or any other storage device or storage disk in which information is stored for any duration (e.g., for extended time periods, permanently, for brief instances, for temporarily buffering, and/or for caching of the information). As used herein, the term tangible computer readable storage medium is expressly defined to include any type of computer readable storage device and/or storage disk and to exclude propagating signals and to exclude transmission media. As used herein, "tangible computer readable storage medium" and "tangible machine readable storage medium" are used interchangeably. Additionally or alternatively, the example process(es) of FIG. 10 may be implemented using coded instructions (e.g., computer and/or machine readable instructions) stored on a non-transitory computer and/or machine readable medium such as a hard disk drive, a flash memory, a read-only memory, a compact disk, a digital versatile disk, a cache, a random-access memory and/or any other storage device or storage disk in which information is stored for any duration (e.g., for extended time periods, permanently, for brief instances, for temporarily buffering, and/or for caching of the information). As used herein, the term non-transitory computer readable medium is expressly defined to include any type of computer readable storage device and/or storage disk and to exclude propagating signals and to exclude transmission media. As used herein, when the phrase "at least" is used as the transition term in a preamble of a claim, it is open-ended in the same manner as the term "comprising" is open ended.

FIG. 10 illustrates a flow diagram of an example method 1000 to monitor receiver and/or other device health in a real time location system. At block 1002, messages are monitored to detect a message from a receiver. For example, the example message receiver 910 monitors for a message from a receiver (e.g., from one or more devices 802-806 including one or more beacons, badges, and/or receivers 608, 610, 612, 614, 708, 710, 712, 714, 716, 718, etc.). At block 1004, when a message is received, the message is evaluated to determine a message type. For example, the example message evaluator 920 evaluates the received message to determine a message type associated with the message (e.g., location message, firmware message, time message, receiver configuration message, health message, etc.). At block 1006, the message type is compared to a health message type. If the message is not a health message, then, at block 1008, the message is redirected for further processing. For example, the message is not a receiver health message, the message evaluator 920 sends the message to another processor, such as the location engine 728, site builder 724, consuming product(s) 732, etc.

If the message is a health message, then, at block 1010, the health message is processed to identify an event type indicated by the health message. For example, the message evaluator 920 sends the message to the event processor 930. The example event processor 930 processes the health message to identify an event type indicated by the message. For example, the message may indicate an on charge event, off charge event, forced reboot event, unforced reboot/system error event, WiFi reconnect event, heartbeat event, etc. At block 1012, based on the event type, the event processor 930 processes the details of the event. For example, the event processor 930 digests information associated with the event type and additional details if provided in the message.

At block 1014, the event is compared to prescribed bound(s) for the event. For example, the event processor 930 provides the message details and event type to the health analyzer. Based on the event type, the example health analyzer 940 compares the details of the event to a threshold, range, standard, norm, etc. At block 1016, if the event is within normal or expected behavior, the event is logged. For example, the event can be logged via the output generator 960. At block 1018, if the event is outside the prescribed bound(s), a response to the event is triggered. For example, the health alert notifier 950 can be triggered in response to the event. In some examples, the health alert notifier 950 can generate a response message or instruction to the device via the output generator 960 to adjust a level, setting, mode, etc., in response to the event (e.g., not charging enough, not charging properly, irregular heartbeat, reboot needed, etc.) such as to send a message to a user, automatically adjust a device setting, trigger a maintenance request, alert hospital staff to a failing device, change in setting/configuration warranted, etc. At block 1020, an output is provided. For example, the output generator 960 can provide an update and/or other message to the device and/or a third party (e.g., beacon/site management services 720, badge configuration services 730, consuming product(s) 732, etc.) to repair, replace, and/or adjust the affected device(s). An alert, update, and/or other message can be generated to help ensure reliable operation and uptime of the RTLS system 600, 700, for example.

FIG. 11 is a block diagram of an example processor platform 1100 capable of executing the instructions of FIG. 10 to implement the example systems and components disclosed and described herein with respect to FIGS. 1-9. The processor platform 1100 can be, for example, a server, a personal computer, or any other type of computing device.

The processor platform 1100 of the illustrated example includes a processor 1112. The processor 1112 of the illustrated example is hardware. For example, the processor 1112 can be implemented by one or more integrated circuits, logic circuits, microprocessors or controllers from any desired family or manufacturer.

The processor 1112 of the illustrated example includes a local memory 1113 (e.g., a cache). The processor 1112 of the illustrated example executes the instructions to implement the example message receiver 910, the example message evaluator 920, the example event processor 930, the example health analyzer 940, the example health alert notifier 950, the example output generator 960, and/or, more generally, the example health processor of FIGS. 6-9. The processor 1112 of the illustrated example is in communication with a main memory including a volatile memory 1114 and a non-volatile memory 1116 via a bus 1118. The volatile memory 1114 may be implemented by Synchronous Dynamic Random Access Memory (SDRAM), Dynamic Random Access Memory (DRAM), RAMBUS Dynamic Random Access Memory (RDRAM) and/or any other type of random access memory device. The non-volatile memory 1116 may be implemented by flash memory and/or any other desired type of memory device. Access to the main memory 1114, 1116 is controlled by a memory controller.

The processor platform 1100 of the illustrated example also includes an interface circuit 1120. The interface circuit 1120 may be implemented by any type of interface standard, such as an Ethernet interface, a universal serial bus (USB), and/or a PCI express interface.

In the illustrated example, one or more input devices 1122 are connected to the interface circuit 1120. The input device(s) 1122 permit(s) a user to enter data and commands into the processor 1112. The input device(s) can be implemented by, for example, an audio sensor, a microphone, a camera (still or video), a keyboard, a button, a mouse, a touchscreen, a track-pad, a trackball, isopoint and/or a voice recognition system.

One or more output devices 1124 are also connected to the interface circuit 1120 of the illustrated example. The output devices 1124 can be implemented, for example, by display devices (e.g., a light emitting diode (LED), an organic light emitting diode (OLED), a liquid crystal display, a cathode ray tube display (CRT), a touchscreen, a tactile output device, a printer and/or speakers). The interface circuit 1120 of the illustrated example, thus, typically includes a graphics driver card, a graphics driver chip or a graphics driver processor.

The interface circuit 1120 of the illustrated example also includes a communication device such as a transmitter, a receiver, a transceiver, a modem and/or network interface card to facilitate exchange of data with external machines (e.g., computing devices of any kind) via a network 1126 (e.g., an Ethernet connection, a digital subscriber line (DSL), a telephone line, coaxial cable, a cellular telephone system, etc.).

The processor platform 1100 of the illustrated example also includes one or more mass storage devices 1128 for storing software and/or data. Examples of such mass storage devices 1128 include floppy disk drives, hard drive disks, compact disk drives, Blu-ray disk drives, RAID systems, and digital versatile disk (DVD) drives.

The coded instructions 1132 of FIG. 10 may be stored in the mass storage device 1128, in the volatile memory 1114, in the non-volatile memory 1116, and/or on a removable tangible computer readable storage medium such as a CD or DVD.

Thus, certain examples communicate via messages or beacon communications to provide status information, location information, firmware upgrade, etc. Certain examples provide a custom beacon protocol (e.g., a custom BLE beacon protocol, etc.) that provides all involved fields for message processing and location determination, as well as system health status including low battery indication (e.g., in days remaining, hours remaining, percent remaining, battery value, etc.), custom fields for additional installation specific data (e.g., profile identifier (ID), floor designation, department ID, etc.), beacon security, and/or other item(s). While no single existing protocol fulfills these needs, certain examples provide a new protocol that enable location systems to provide customers with system health and security information/messaging and also enable additional device level functionality by allowing custom data within the protocol. Certain examples provide a protocol including data encryption as well as battery life information, system health information, location information, and/or other custom information. Using this protocol, the health processor 830, management services 840, edge device 810, location gateway 618, location server 620, badge configuration tool 702, firmware 704, location toolbox app 706, beacon/site management services 720, site builder 724, GIS 726, location engine 728, badge configuration services 730, consuming products 732, etc., can communicate and use the protocol to transmit and/or receive a plurality of information.

For example, a BLE proximity-sensing protocol can transmit a UUID along with several additional bytes to determine a device's physical location, track device movement, trigger a location-based action on the device, gauge device health, update device firmware, etc., via push and/or pull communication. Certain examples provide a protocol with a plurality of frame types that can be used individually and/or in various combinations to communication information from a beacon to a receiver. For example, a frame can include an identifier, a type, a flag, a length, a data value, an encrypted value, etc. A frame can include a variable data value and an indication of how the receiver is to interpret that value.

Certain examples can specify a security type (e.g., WPA-Enterprise, etc.), an encryption type (e.g., TKIP, etc.), an authentication method (e.g., Cisco: PEAP, etc.), authentication credentials (e.g., username/password, certificate, etc.), gateway host, gateway port, etc. Profile and proximity range can also be defined, for example. Configuration setting and corresponding BLE channel(s) can also be defined, for example. Other parameters such as location beacon broadcast, badge scan profile indicator, proximity range identifier, transmit interval, transmit window, hit count, suppress repetitive, etc., can be defined according to the protocol, for example.

From the foregoing, it will appreciate that the above disclosed methods, apparatus and articles of manufacture facilitate proximity detection and location tracking of assets in an industrial setting. As described above, example disclosures uniquely eliminate the expensive and difficult-to-maintain infrastructure. An example benefit of the disclosed techniques includes determining location awareness of assets in the industrial setting without constructing a new infrastructure. In some disclosed examples, the location awareness of assets is determined by "crowd-sourcing" probability proximity locations of the assets.

Although certain example methods, apparatus and articles of manufacture have been disclosed herein, the scope of coverage of this patent is not limited thereto. On the contrary, the invention is defined by the claims.

## Claims

1. A cloud based processing system (830) configured to:
receive location information and health status information of a plurality of beacon devices, wherein the location information indicates real-time locations of the plurality of beacon devices, and the health status information indicates real-time conditions of the plurality of beacon devices, the health status information including an event associated with at least one of the real-time conditions occurring at the respective beacon device, wherein the event comprises operation error information including one of: an unforced reboot event, a system error event, a forced reboot event, a wi-fi reconnect event and a heartbeat event, and wherein transmission of the health status information is in response to occurrence of the condition at the respective device;
track movements of at least some of the plurality of beacon devices based on the location information; and **characterized in that** the system is configured to compare details of the event to a prescribed bound (1014), and, if the event is outside the prescribed bound, trigger a response (1018) to the event, wherein the response comprises outputting an update or other message to repair, replace, and/or adjust the device.

2. The cloud based processing system of claim 1, wherein the plurality of beacon devices comprises beacon tags (405) installed in or attached to fixed location assets, beacon tags affixed to mobile assets, and reader badgers (425) worn by healthcare workers (426).

3. The cloud based processing system of claim 1, wherein the location information and health status information are transmitted as fields in frames following a custom beacon communication protocol.

4. The cloud based processing system of claim 3, wherein the custom beacon communication protocol facilitates Bluetooth Low Energy (BLE) communication.

5. The cloud based processing system of claim 3, wherein the custom beacon communication protocol also provides fields for installation specific data.

6. The cloud based processing system of claim 3, wherein the custom beacon protocol also provides fields for beacon security information.

7. A method performed by a cloud based processing system (830), the method comprising:
receiving location information and health status information of a plurality of beacon devices, wherein the location information indicates real-time locations of the plurality of beacon devices, and the health status information indicates real-time conditions of the plurality of beacon devices, the health status information including an event associated with at least one of the real-time conditions occurring at the respective beacon device, wherein transmission of the health status information is in response to occurrence of the condition at the respective device;
tracking movements of at least some of the plurality of beacon devices based on the location information; and **characterized in that** the method comprises comparing details of the event to a prescribed bound (1014), and, if the event is outside the prescribed bound, triggering a response (1018) to the event, wherein the event comprises operation error information including one of: an unforced reboot event, a system error event, a forced reboot event, a wi-fi reconnect event and a heartbeat event, and wherein the response comprises outputting an update or other message to repair, replace, and/or adjust the device.

8. The method of claim 7, wherein the plurality of beacon devices comprises beacon tags (405) installed in or attached to fixed location assets, beacon tags affixed to mobile assets, and reader badgers (425) worn by healthcare workers.

9. The method of claim 7, wherein the location information and health status information are transmitted as fields in frames following a custom beacon communication protocol.

10. The method of claim 9, wherein the custom beacon communication protocol facilitates Bluetooth Low Energy (BLE) communication.

11. The method of claim 9, wherein the custom beacon communication protocol also provides fields for installation specific data.

12. The method of claim 9, wherein the custom beacon protocol also provides fields for beacon security information.

13. A non-transitory computer readable storage medium including instructions, when executed, cause a processing system to perform operations of:
receiving location information and health status information of a plurality of beacon devices, wherein the location information indicates real-time locations of the plurality of beacon devices, and the health status information indicates real-time conditions of the plurality of beacon devices, the health status information including an event associated with at least one of the real-time conditions occurring at the respective beacon device, wherein transmission of the health status information is in response to occurrence of the condition at the respective device;
tracking movements of at least some of the plurality of beacon devices based on the location information; and **characterized in that** the instructions, when executed, further cause the processing system to perform operations of:
comparing details of the event to a prescribed bound (1014), and, if the event is outside the prescribed bound, trigger a response (1018) to the event, wherein the event comprises operation error information including one of: an unforced reboot event, a system error event, a forced reboot event, a wi-fi reconnect event and a heartbeat event, and wherein the response comprises outputting an update or other message to repair, replace, and/or adjust the device.

## Patentansprüche

1. Cloud-basiertes Verarbeitungssystem (830), das konfiguriert ist zum:
Empfangen von Standortinformationen und Gerätezustandsinformationen einer Vielzahl von Beaconvorrichtungen, wobei die Standortinformationen Echtzeit-Standorte der Vielzahl von Beaconvorrichtungen anzeigen, und wobei die Gerätezustandsinformationen Echtzeit-Bedingungen der Vielzahl von Beaconvorrichtung anzeigen, wobei die Gerätezustandsinformationen ein Ereignis beinhalten, das mit mindestens einer der Echtzeit-Bedingungen assoziiert ist, die in der entsprechenden Beaconvorrichtung auftreten, wobei das Ereignis Betriebsfehlerinformationen umfasst, die einen beinhalten von: einem unerzwungenen Neustartereignis, einem Systemfehlerereignis, einem erzwungenen Neustartereignis, einem Wi-Fi-Neuverbindungsereignis und einem Heartbeat-Ereignis, und wobei eine Übertragung der Gerätezustandsinformationen als Reaktion auf ein Auftreten der Bedingung in der entsprechenden Vorrichtung erfolgt;
Verfolgen von Bewegungen von mindestens einigen aus der Vielzahl von Beaconvorrichtungen basierend auf den Standortinformationen; und **dadurch gekennzeichnet, dass** das System konfiguriert ist zum Vergleichen von Einzelheiten des Ereignisses mit einer vorgeschriebenen Grenze (1014), und, wenn das Ereignis außerhalb der vorgeschriebenen Grenze auftritt, Auslösen einer Reaktion (1018) auf das Ereignis, wobei die Reaktion ein Ausgeben einer Aktualisierung oder einer anderen Nachricht umfasst, um die Vorrichtung zu reparieren, zu ersetzen und/oder anzupassen.

2. Cloud-basiertes Verarbeitungssystem nach Anspruch 1, wobei die Vielzahl von Beaconvorrichtungen umfasst: Beacontags (405), die in Standorteinrichtungen installiert sind oder daran befestigt sind; Beacontags die an mobilen Einrichtungen befestigt sind; und Lesegerät-Badgers (425), die von Mitarbeitern des Gesundheitswesens (426) getragen werden.

3. Cloud-basiertes Verarbeitungssystem nach Anspruch 1, wobei die Standortinformationen und die Gerätezustandsinformationen als Felder in Frames übertragen werden, die ein kundenspezifisches Beaconkommunikationsprotokoll befolgen.

4. Cloud-basiertes Verarbeitungssystem nach Anspruch 3, wobei das kundenspezifische Beaconkommunikationsprotokoll eine Niedrigenergie-Bluetooth-Kommunikation (BLE-Kommunikation) vereinfacht.

5. Cloud-basiertes Verarbeitungssystem nach Anspruch 3, wobei das kundenspezifische Beaconkommunikationsprotokoll auch Felder für installationsspezifische Daten bereitstellt.

6. Cloud-basiertes Verarbeitungssystem nach Anspruch 3, wobei das kundenspezifische Beaconkommunikationsprotokoll auch Felder für Beaconsicherheitsinformationen bereitstellt.

7. Verfahren, das von einem Cloud-basierten Verarbeitungssystem (830) durchgeführt wird, wobei das Verfahren umfasst:
Empfangen von Standortinformationen und Gerätezustandsinformationen einer Vielzahl von Beaconvorrichtungen, wobei die Standortinformationen Echtzeit-Standorte der Vielzahl von Beaconvorrichtungen anzeigen, und wobei die Gerätezustandsinformationen Echtzeit-Bedingungen der Vielzahl von Beaconvorrichtung anzeigen, wobei die Gerätezustandsinformationen ein Ereignis beinhalten, das mit mindestens einer der Echtzeit-Bedingungen assoziiert ist, die in der entsprechenden Beaconvorrichtung auftreten, wobei eine Übertragung der Gerätezustandsinformationen als Reaktion auf ein Auftreten der Bedingung in der entsprechenden Vorrichtung erfolgt;
Verfolgen von Bewegungen von mindestens einigen aus der Vielzahl von Beaconvorrichtungen basierend auf den Standortinformationen; und **dadurch gekennzeichnet, dass** das Verfahren umfasst: Vergleichen von Einzelheiten des Ereignisses mit einer vorgeschriebenen Grenze (1014); und, wenn das Ereignis außerhalb der vorgeschriebenen Grenze auftritt, Auslösen einer Reaktion (1018) auf das Ereignis, wobei das Ereignis Betriebsfehlerinformationen umfasst, die einen beinhalten von: einem unerzwungenen Neustartereignis, einem Systemfehlerereignis, einem erzwungenen Neustartereignis, einem Wi-Fi-Neuverbindungsereignis und einem Heartbeat-Ereignis, und wobei die Reaktion ein Ausgeben einer Aktualisierung oder einer anderen Nachricht umfasst, um die Vorrichtung zu reparieren, zu ersetzen und/oder anzupassen.

8. Verfahren nach Anspruch 7, wobei die Vielzahl von Beaconvorrichtungen umfasst: Beacontags (405), die in Standorteinrichtungen installiert sind oder daran befestigt sind; Beacontags die an mobilen Einrichtungen befestigt sind; und Lesegerät-Badgers (425), die von Mitarbeitern des Gesundheitswesens getragen werden.

9. Verfahren nach Anspruch 7, wobei die Standortinformationen und Gerätezustandsinformationen als Felder in Frames übertragen werden, die ein kundenspezifisches Beaconkommunikationsprotokoll befolgen.

10. Verfahren nach Anspruch 9, wobei das kundenspezifische Beaconkommunikationsprotokoll eine Niedrigenergie-Bluetooth-Kommunikation (BLE-Kommunikation) vereinfacht.

11. Verfahren nach Anspruch 9, wobei das kundenspezifische Beaconkommunikationsprotokoll auch Felder für installationsspezifische Daten bereitstellt.

12. Verfahren nach Anspruch 9, wobei das kundenspezifische Beaconkommunikationsprotokoll auch Felder für Beaconsicherheitsinformationen bereitstellt.

13. Nicht-flüchtiges computerlesbares Speichermedium, das Anweisungen beinhaltet, die, wenn sie ausgeführt werden, ein Verarbeitungssystem veranlassen, die folgenden Operationen durchzuführen:
Empfangen von Standortinformationen und Gerätezustandsinformationen einer Vielzahl von Beaconvorrichtungen, wobei die Standortinformationen Echtzeit-Standorte der Vielzahl von Beaconvorrichtungen anzeigen, und wobei die Gerätezustandsinformationen Echtzeit-Bedingungen der Vielzahl von Beaconvorrichtung anzeigen, wobei die Gerätezustandsinformationen ein Ereignis beinhalten, das mit mindestens einer der Echtzeit-Bedingungen assoziiert ist, die in der entsprechenden Beaconvorrichtung auftreten, wobei eine Übertragung der Gerätezustandsinformationen als Reaktion auf ein Auftreten der Bedingung in der entsprechenden Vorrichtung erfolgt;
Verfolgen von Bewegungen von mindestens einigen aus der Vielzahl von Beaconvorrichtungen basierend auf den Standortinformationen; und **dadurch gekennzeichnet, dass** die Anweisungen, wenn sie ausgeführt werden, ein Verarbeitungssystem ferner veranlassen, die folgenden Operationen durchzuführen:
Vergleichen von Einzelheiten des Ereignisses mit einer vorgeschriebenen Grenze (1014); und, wenn das Ereignis außerhalb der vorgeschriebenen Grenze auftritt, Auslösen einer Reaktion (1018) auf das Ereignis, wobei das Ereignis Betriebsfehlerinformationen umfasst, die einen beinhalten von: einem unerzwungenen Neustartereignis, einem Systemfehlerereignis, einem erzwungenen Neustartereignis, einem Wi-Fi-Neuverbindungsereignis und einem Heartbeat-Ereignis, und wobei die Reaktion ein Ausgeben einer Aktualisierung oder einer anderen Nachricht umfasst, um die Vorrichtung zu reparieren, zu ersetzen und/oder anzupassen.

## Revendications

1. Système de traitement basé sur le cloud (830) configuré pour :
recevoir des informations de localisation et des informations d'état de santé d'une pluralité de dispositifs balises, où les informations de localisation indiquent les emplacements en temps réel de la pluralité de dispositifs balises, et les informations d'état de santé indiquent les conditions en temps réel de la pluralité de dispositifs balises, les informations sur l'état de santé comprenant un événement associé à au moins l'une des conditions en temps réel se produisant au niveau du dispositif balise respectif, où l'événement comprend des informations sur une erreur de fonctionnement comprenant l'un des éléments suivants :
un événement de redémarrage non forcé, un événement d'erreur système, un événement de redémarrage forcé, un événement de reconnexion Wi-Fi et un événement de pulsation, et où la transmission des informations sur l'état de santé se fait en réponse à la survenue de la condition au niveau du dispositif respectif ;
suivre les mouvements d'au moins certains des dispositifs balises sur la base des informations de localisation ;
et **caractérisé en ce que** le système est configuré pour comparer les détails de l'événement à une limite prescrite (1014) et, si l'événement se situe en dehors de la limite prescrite, déclencher une réponse (1018) à l'événement, la réponse comprenant l'envoi d'une mise à jour ou d'un autre message pour réparer, remplacer et/ou ajuster le dispositif.

2. Système de traitement basé sur le cloud selon la revendication 1, dans lequel la pluralité de dispositifs balises comprend des balises (405) installées dans, ou fixées à, des actifs à emplacement fixe, des balises fixées à des actifs mobiles et des badges de lecture (425) portés par des professionnels de santé (426).

3. Système de traitement basé sur le cloud selon la revendication 1, dans lequel les informations de localisation et les informations sur l'état de santé sont transmises sous forme de champs dans des trames suivant un protocole de communication de balise personnalisé.

4. Système de traitement basé sur le cloud selon la revendication 3, dans lequel le protocole de communication de balise personnalisé facilite la communication Bluetooth Low Energy (BLE).

5. Système de traitement basé sur le cloud selon la revendication 3, dans lequel le protocole de communication de balise personnalisé fournit également des champs pour les données spécifiques à l'installation.

6. Système de traitement basé sur le cloud selon la revendication 3, dans lequel le protocole de balise personnalisé fournit également des champs pour des informations de sécurité de balise.

7. Procédé mis en œuvre par un système de traitement basé sur le cloud (830), le procédé comprenant :
la réception d'informations de localisation et d'informations d'état de santé d'une pluralité de dispositifs balises, où les informations de localisation indiquent les emplacements en temps réel de la pluralité de dispositifs balises, et les informations d'état de santé indiquent les conditions en temps réel de la pluralité de dispositifs balises, les informations sur l'état de santé comprenant un événement associé à au moins l'une des conditions en temps réel se produisant au niveau du dispositif balise respectif, où la transmission des informations sur l'état de santé se fait en réponse à la survenue de la condition au niveau du dispositif respectif ;
le suivi des mouvements d'au moins certains de la pluralité de dispositifs de balisage sur la base des informations de localisation ; le procédé étant **caractérisé en ce qu'**il comprend la comparaison des détails de l'événement à une limite prescrite (1014) et, si l'événement se trouve en dehors de la limite prescrite, le déclenchement d'une réponse (1018) à l'événement, où l'événement comprend des informations d'erreur de fonctionnement comprenant l'un des éléments suivants : un événement de redémarrage non forcé, un événement d'erreur système, un événement de redémarrage forcé, un événement de reconnexion Wi-Fi et un événement de pulsation, et où la réponse comprend la sortie d'une mise à jour ou d'un autre message pour réparer, remplacer et/ou ajuster le dispositif.

8. Procédé selon la revendication 7, dans lequel la pluralité de dispositifs balises comprend des balises (405) installées dans, ou fixées à, des actifs à emplacement fixe, des balises fixées à des actifs mobiles et des badges de lecture (425) portés par des professionnels de santé.

9. Procédé selon la revendication 7, dans lequel les informations de localisation et les informations sur l'état de santé sont transmises sous forme de champs dans des trames suivant un protocole de communication de balise personnalisé.

10. Procédé selon la revendication 9, dans lequel le protocole de communication de balise personnalisé facilite la communication Bluetooth Low Energy (BLE).

11. Procédé selon la revendication 9, dans lequel le protocole de communication de balise personnalisé fournit également des champs pour les données spécifiques à l'installation.

12. Procédé selon la revendication 9, dans lequel le protocole de balise personnalisé fournit également des champs pour des informations de sécurité de balise.

13. Support de stockage non transitoire lisible par ordinateur comprenant des instructions qui, lorsqu'elles sont exécutées, amènent un système de traitement à effectuer les opérations suivantes :
la réception d'informations de localisation et d'informations d'état de santé d'une pluralité de dispositifs balises, où les informations de localisation indiquent les emplacements en temps réel de la pluralité de dispositifs balises, et les informations d'état de santé indiquent les conditions en temps réel de la pluralité de dispositifs balises, les informations sur l'état de santé comprenant un événement associé à au moins l'une des conditions en temps réel se produisant au niveau du dispositif balise respectif, où la transmission des informations sur l'état de santé se fait en réponse à la survenue de la condition au niveau du dispositif respectif ;
le suivi des mouvements d'au moins certains des dispositifs balises sur la base des informations de localisation ; et **caractérisé en ce que** les instructions, lorsqu'elles sont exécutées, amènent en outre le système de traitement à effectuer les opérations suivantes :
la comparaison des détails de l'événement à une limite prescrite (1014) et, si l'événement se trouve en dehors de la limite prescrite, le déclenchement d'une réponse (1018) à l'événement, où l'événement comprend des informations d'erreur de fonctionnement comprenant l'un des éléments suivants : un événement de redémarrage non forcé, un événement d'erreur système, un événement de redémarrage forcé, un événement de reconnexion Wi-Fi et un événement de pulsation, et où la réponse comprend la sortie d'une mise à jour ou d'un autre message pour réparer, remplacer et/ou ajuster le dispositif.
